# EUROPÄISCHE PATENTSCHRIFT

(11) **EP 3 164 696 B1**
(45) Veröffentlichungstag und Bekanntmachung des Hinweises auf die Patenterteilung: **18.04.2018**
(21) Anmeldenummer: 15759358.3
(22) Anmeldetag: 03.07.2015
(51) Int. Cl.: G01N 21/65, G01N 33/08, G01N 33/483

(54) **VERFAHREN UND VORRICHTUNG ZUR RAMANSPEKTROSKOPISCHEN IN-OVO GESCHLECHTSBESTIMMUNG VON BEFRUCHTETEN UND BEBRÜTETEN VOGELEIERN**
METHOD AND DEVICE FOR RAMAN SPECTROSCOPIC IN-OVO SEX DETERMINATION OF FERTILIZED AND INCUBATED BIRD EGGS
PROCÉDÉ ET DISPOSITIF POUR LA DÉTERMINATION IN-OVO DU SEXE D'OEUFS D'OISEAUX FERTILISÉS ET COUVÉS PAR SPECTROSCOPIE RAMAN

(30) Priorität: 04.07.2014 DE 102014010150
(43) Veröffentlichungstag der Anmeldung: 10.05.2017
(73) Patentinhaber: Technische Universität Dresden, 01069 Dresden (DE); Universität Leipzig, 04109 Leipzig (DE)
(72) Erfinder: GALLI, Roberta, 01309 Dresden (DE); PREUSSE, Grit, 01445 Radebeul (DE); KOCH, Edmund, 01307 Dresden (DE); STEINER, Gerald, 08340 Schwarzenberg (DE); KRAUTWALD-JUNGHANNS, Maria-Elisabeth, 04416 Markleeberg (DE); BARTELS, Thomas, 30989 Gehrden (DE)
(74) Vertreter: Rauschenbach, Marion
(86) Internationale Anmeldenummer: PCT/DE2015/000342
(87) Internationale Veröffentlichungsnummer: WO 2016/000678

(56) Entgegenhaltungen:
- WO-A2-2014/021715
- DE-A1-102007 013 107
- M. Harz ET AL: "Minimal Invasive Gender Determination of Birds by Means of UV-Resonance Raman Spectroscopy", Analytical Chemistry, vol. 80, no. 4, 1 February 2008 (2008-02-01), pages 1080-1086, XP055422385, US ISSN: 0003-2700, DOI: 10.1021/ac702043q

## Beschreibung

Die Erfindung betrifft ein Verfahren und eine Vorrichtung zur Ramanspektroskopischen in-ovo Geschlechtsbestimmung von befruchteten und bebrüteten Vogeleiern.

In der Druckschrift DE 10 2010 006 161 B3 sind ein Verfahren und eine Vorrichtung zur Bestimmung des Geschlechts von befruchteten und nicht bebrüteten Vogeleiern beschrieben, wobei ein Ei zumindest eine feste Kalkschale, ein von der Kalkschale und weiteren Eihüllen umgebenes Eidotter und eine dem Eidotter zugeordnete Keimscheibe enthält, wobei eine Sonde zur Messung eines Spektrums durch ein Loch der Kalkschale hindurch in Richtung zur Keimscheibe mit Keimscheibenzellen geführt wird,
wobei das Verfahren folgende Schritte aufweist:
- Positionierung der Sonde im Bereich der Keimscheibe,
- spektroskopische in-ovo Charakterisierung der Keimscheibenzellen,
- Erkennung des Geschlechts durch eine automatische Klassifizierung der reflektiven Spektren,
wobei als Sonde ein optischer Kristall eingesetzt wird, mit dem eine schnelle und rückwirkungsfreie Aufnahme eines Infrarot- und/oder Nahinfrarotspektrums bei Nutzung der abgeschwächten Totalreflexion innerhalb des optischen Kristalls durch das evaneszente Feld im Bereich der Keimscheibe durchgeführt wird,
wobei die Extinktion infolge einer spektralen Absorption von geschlechtsspezifischen Keimscheibenzellen erfolgt,
wobei die Positionierung des optischen Kristalls durch eine permanente automatische Auswertung der rückgeführten Spektren bis zur Bestimmung von geschlechtsspezifischen Keimscheibenzellen begleitet wird, bis das Spektrum ausgewertet und das Geschlecht des befruchteten Eies eindeutig angezeigt wird.

Während des Positionierungsvorgangs werden permanent rückgeführte IR- und/oder NIR-Spektren aufgezeichnet und einer Auswertung zugeführt, wobei eine automatische Klassifizierung der Spektren anhand des spektralen Fingerabdruckes zum Beispiel in Proteinen, Lipiden und Nukleinsäuren erfolgt.

Bei der geschlechtsspezifischen Absorption des einfallenden IR- und/oder NIR-Lichts werden die Keimscheibenzellen anhand von Absorptionsbanden der Nukleinsäuren (DNA und RNA) sowie weiterer biochemischer Verbindungen derart identifiziert, dass das Geschlecht des geprüften Eies bestimmt und angezeigt wird.

Die Messung kann mit einer herkömmlichen Infrarot-Spektroskopie durchgeführt werden.

Die in der Druckschrift DE 10 2010 006 161 B3 beschriebene zugehörige Vorrichtung enthält
- mindestens eine Eipositions-Auflage zur Arretierung mindestens eines Eies,
- mindestens eine Höhenverstelleinrichtung mit mindestens einem Haltearm,
- mindestens einen als Sonde ausgebildeten optischen Kristall, der an dem Haltearm befestigt ist,
- mindestens eine Steuereinheit für die eiarretierende Eipositions-Auflage und für die Höhenverstelleinrichtung,
- mindestens eine auf mindestens einen Wellenlängenbereich bezogene spektrale Lichtquelle, die einen IR- und/oder NIR-Lichtstrahl aussendet,
- mindestens einen Detektor zur Aufnahme des rückgeführten IR- und/oder NIR-Lichtstrahls,
- mindestens ein optisches Element zur geführten Strahlführung zwischen der Lichtquelle und dem optischen Kristall und zur rückgeführten Strahlführung vom optischen Kristall hin zum Detektor, sowie
- eine mit dem Detektor verbundene Auswerteeinheit und eine Anzeigeeinheit,
wobei mit der Höhenverstelleinrichtung die Höhe des Haltearms und somit des optischen Kristalls in Bezug auf den Ort der Keimscheibe einstellbar ist und der optischen Kristall im Bereich der Keimscheibe in einer Scheibenzuordnungsposition positionierbar ist, in der über den optischen Kristall ein sich bei Totalreflexion an der zur Keimscheibe gerichteten Ausgangsfläche ausbildendes evaneszentes Feld auf die Keimscheibe übergreift und die darin befindlichen Keimscheibenzellen wechselwirkend mit dem evaneszenten Feld eine geschlechtsspezifische Absorption des Licht aus dem einfallenden Strahlengang vornehmen, wobei das an der Ausgangsfläche totalreflektierte Licht über den rückgeführten Strahlengang innerhalb des Kristalls und schließlich über das optische Element zur Registrierung zum Detektor geführt ist, von dem aus die registrierten spektralen Signale zur Auswertung und Anzeige des Geschlechts übermittelt werden.

In der Druckschrift DE 10 2007 013 107 A1 wird ein DNA relevantes Zellmaterial durch Schwingungsspektroskopie analysiert, um das Geschlecht von Vogeleiern anhand von DNA-Unterschieden zu bestimmen: Entweder im Ei nach Öffnung mit einer Sonde oder nach Entnahme des Materials aus dem Ei und Deposition auf einem Substrat. Die UV-Resonanzraman-Spektroskopie wird bei Wellenlängen von 244 nm oder 254 nm festgelegt. Dabei wird eine Spektrenklassifikation mit allen existierenden überwachten und nichtüberwachten Verfahren durchgeführt:
- mit einem DNA-relevanten Material, mit speziellem Bezug zur Federpulpa und der Keimscheibe und
- einer UV-Raman-Spektroskopie bei einer Wellenlänge von 244 nm oder 257 nm.

Bei diesem Verfahren wird somit DNA-relevantes Zellmaterial des geschlechtlich zu bestimmenden Vogels mit Licht untersucht und die Molekülschwingungen gemessen, wobei das durch das Licht entstehende Spektrum der Molekülschwingungen erfasst und mit vorgegebenen sowie geschlechtsspezifische DNA-Strukturen der zu untersuchenden Vogelart repräsentierenden Referenzspektren verglichen wird und wobei aus diesem Spektralvergleich eine auf Grundlage des DNA-Gehalts des Zellmaterials basierende Geschlechtszuordnung des Vogels getroffen wird.

Die Molekülschwingungen werden dabei mittels Anwendung der Raman-Spektroskopie oder der IR-Spektroskopie gemessen, wobei z.B. das DNArelevante Zellmaterial aus dem Schaft einer jungen Feder eines Vogels entnommen werden kann. Das Zellmaterial wird auf einem Träger präpariert und mit Licht abgetastet.

In einem anderen in der Druckschrift DE 10 2007 013 107 A1 beschriebenen Teilverfahren wird das Licht zur Messung der Molekülschwingungen des DNA-relevanten Zellmaterials von ungeschlüpften Vögeln durch die Eierschale hindurch auf den Embryo oder die Keimscheibe fokussiert, wobei das Spektrum der durch die Molekülschwingungen entstehenden Strahlung im Ei mit einer durch dessen Schale hindurch geführten Sonde gemessen wird.
Für die Hindurchführung der Sonde wird zur Messung des Spektrums wenigstens ein mikroskopisch kleines Loch durch die Eischale hindurch gebohrt. Das Licht wird durch den kleinen Zugang durch die Eischale unmittelbar auf die Keimscheibe als Zellmaterial fokussiert. Durch den gleichen oder einen anderen Zugang geringer Öffnungsgröße wird die Sonde eingeführt, mittels der das reflektierte und von der Sonde aufgenommene Spektrum der vorgenannten Molekülbewegung im Innern des Eis gemessen wird.

Die erhaltenen spektralen Informationen werden in einem zweiten Schritt mit geschlechtsspezifischen Referenzdaten verglichen und einem Klassifizierungsalgorithmus zugeführt. Diese repräsentieren vorzugsweise statistisch gewonnene Daten über die zu untersuchenden Vogelspezies. Aus diesem Vergleich wird die Geschlechtszuordnung des zu untersuchenden DNA-Materials getroffen.

Ein Problem besteht darin, dass für die Einbringung einer Sonde in vorgefertigte Löcher bei einer sehr großen Anzahl von zu untersuchenden Vogeleiern ein großer zeitlicher Aufwand erforderlich ist. Außerdem muss bei einer Fokussierung des Lichts aus der Sonde auf die Keimscheibe ein erheblicher Justierungsaufwand für eine optische Abbildung in Bezug auf den Ort der Keimscheibe betrieben werden, wobei von Ei zu Ei die Fokussierungsebene eine andere Lage aufweisen kann und damit keine Bestimmung des Geschlechts durchgeführt werden kann.

Problematisch an diesen Untersuchungen ist weiterhin, dass das zur Untersuchung der Keimscheiben notwendige Einbringen der Löcher in die Kalkschale einschließlich der Eischalenmembran am Tag "Null" zu einer Beeinträchtigung der Emryonalentwicklung und zu stark sinkenden Schlupfraten führt, wie in den Druckschriften S. Klein: Analysis of chicken embryonic development after removal of blastodermal cells for sexing. British Poultry Science (39), 1998, S. 482-487; einschließlich darin zitierter Literatur: J. Brake, T. W. (1997). Egg handling and storage. Poultry science (76), S. 144-151 beschrieben ist.

In der Druckschrift WO 2014/021715 A2 wird eine Geschlechtsbestimmung von Vogelembryonen beschrieben, wobei das Verfahren durchgeführt wird mittels
a) Detektion einer Markerverbindung von Zuckern und Aminosäuren, Vorstufen und Metaboliten in der Allantoisflüssigkeit des Eies am 8.-11. Bruttag,
b) Quantitative Bestimmung des Markers mittels NMR-Spektroskopie,
c) Geschlechtsbestimmung durch Vergleich der Menge des Markers zu einem vorgegebenen Ausgangswert.

Dabei erfolgt eine
- Bestimmung der absoluten Mengen oder von Mengenverhältnissen von Verbindungen (Glukose, Cholin, Valin), um sie mit einem Basiswert zu vergleichen,
- Anwendung von unsupervised chemometrischen Verfahren (wie leastsquare modeling oder PCA) auf quantitative Mengen oder Mengenverhältnissen zur Geschlechtsbestimmung.
Ein wesentlicher Nachteil des Verfahrens besteht darin, dass eine Entnahme mindestens einer Probe aus dem Ei erfolgt.

In den Druckschriften US 8 364 247 B2 und EP 2 336 751 A1 wird ein Verfahren zur Bestimmung des Geschlechts an Vogeleiern beschrieben, bei dem mit einer Strahlungsquelle elektromagnetische Strahlung auf die Keimscheibe eines Eies emittiert und nach dem Abschalten der Strahlungsquelle am bestrahlten Bereich der Keimscheibe das Abklingverhalten der angeregten Eigenfluoreszenzintensität zeit- und spektralaufgelöst für mindestens eine Wellenlänge der Eigenfluoreszenz mit einem Detektor erfasst wird. Mit den ermittelten Intensitätsmesswerten wird die fraktale Dimension berechnet und der Wert der fraktalen Dimension DF mit einem art- und geschlechtsspezifischen Grenzwert verglichen; wobei bei Überschreiten des Grenzwertes das jeweilige Ei als weiblich und bei Unterschreiten als männlich eingestuft wird.
Die Ei-Öffnung am Tag "Null" führt zu stark reduzierten Schlupfraten. Es besteht weiterhin eine mögliche irreparable Schädigung der Keimscheibe durch die eingesetzte UV-Strahlung bei 337nm.

In der Druckschrift US 7 950 349 B1 wird ein Verfahren zur Bestimmung:
1) der Fruchtbarkeit eines Vogeleies durch Messung der Lumineszenz und der Biophotonenintensität (Photonen pro Sekunde) des Eies nach Einwirkung einer externen Lichtquelle und
2) des Geschlechtes eines Vogeleies durch Messen des Photonenspektrums der Biophotonenemission und Lumineszenz des Eies nach Einwirkung einer externen Lichtquelle
beschrieben.
Dabei kann die externe Lichtquelle entweder eine Glühlampe, Leuchtstofflampe, LED oder eine (gepulste oder CW) monochromatische oder dichromatische Laserlichtquelle sein. Der erste Teil des Verfahrens ergibt sich aus der Tatsache, dass nach Exposition mit der Lichtquelle, die fruchtbaren Vogeleier eine höhere Intensität der Photonen als die der unbefruchteten Vogeleier emittieren. Der zweite Teil des Verfahrens ergibt sich aus der Tatsache, dass nach Exposition mit den genannten Lichtquellen, Vogeleier des weiblichen Geschlechts ein anderes Spektrum von Photonen als Vogeleier des männlichen Geschlechts emittieren.

In den Druckschriften WO 2010/103111 A1 und US 2012/0058052 A1 werden ein nicht-invasives Verfahren und eine Vorrichtung zur in-ovo-Bestimmung des Geschlechts von Vogelarten beschrieben. Das Verfahren umfasst die Schritte der Einführung eines markierten Antikörpers in das Ei, der sich an ein geschlechtsspezifisches Antigen des Embryos anbindet, und die Detektion des markierten gebundenen Antikörpers mit einer Erfassungseinrichtung außerhalb des Eies.

In der Druckschrift US 7 041 439 B2 werden ein Verfahren und eine Vorrichtung für die automatisierte Prozessführung von Eiern nach ausgewählten Charakteristika (z.B. des Geschlechtes) beschrieben, wobei folgende Schritte ablaufen:
a) Extraktion von Probenmaterial (Allantoisflüssigkeit, Eiweiß, Eigelb, Eischale, Albumin, Gewebe, Membran und / oder Blut),
b) Analyse des extrahierten Materials zur Bestimmung der ausgewählten Charakteristika und
c) selektive Prozessführung der identifizierten Eier.
Beispielsweise wird darin ein Verfahren zur Verarbeitung von Eiern auf der Basis des Geschlechts dargestellt, welches aus folgenden Schritten besteht:
1) Identifizierung lebender Eier,
2) Extraktion von Allantoisflüssigkeit aus den als lebend identifizierten Eiern,
3) Ermittlung des Ostrogengehaltes und einer Farbveränderung in der extrahierten Allantois-Flüssigkeit zur Geschlechtserkennung,
4) selektive Injektion eines Impfstoffs in Abhängigkeit des Geschlechtes.
Ein Nachteil besteht darin, dass die Untersuchung der Allantoisflüssigkeit am Tag 13 bis 18 erfolgt. Auch hier ist eine Probenentnahme notwendig.

In der Druckschrift US 6 365 339 B1 ist ein Verfahren zur Geschlechtsbestimmung von Vogelembryonen beschrieben, bei dem während des Brutprozesses nach Aufbohren der Kalkschale Proben von der Allantoisflüssigkeit des Embryos genommen und in einem lonenmobilitätsspektrometer (IMS) analysiert werden. Die resultierenden Spektren enthalten relevante Markerpeaks, die mit geschlechtsspezifischen Mobilitäten korrelieren.
Ein Nachteil besteht darin, dass auch hier Proben entnommen werden müssen, die einen höheren Aufwand zumindest im Umfeld zur Geschlechtsbestimmung bedingen.

In den Druckschriften WO 2010/150265 A3 und US 2013/0044210 A1 wird ein Verfahren zur Geschlechtsbestimmung unbebrüteter Vogeleier durch hyperspektrale Analyse optischer Spektren (bevorzugt Reflexionsspektren) beschrieben. Die Analyse erfolgt in einem Spektralbereich mit Wellenlängen bis zu 2500 nm (MIR), um das vom CaCO3 der Kalkschale des Eies erzeugte Signal bei 2340 nm herausfiltern zu können. Das Verfahren ermöglicht es, biologische Komponenten, andere als Blut, zu detektieren und ermöglicht sowohl die Erfassung der Fruchtbarkeit vor dem zweiten Bebrütungstag als auch die Bestimmung des Geschlechts der Küken im Ei am zwölften Bebrütungstag. Die Sensitivität konnte durch die Verwendung einer neuronalen Netzwerkanalyse erhöht werden. Mittels der Hauptkomponentenanalyse (PCA) werden die spektralen Merkmale bestimmt, die für die Varianzen zwischen den unbefruchteten Kontrolleiern und den Probeneiern verantwortlich sind. Mittels einer neuronalen Netzwerkanalyse auf der Grundlage der PCA-Ergebnisse werden dann die kleinen, aber signifikanten Veränderungen zwischen den Kontroll- und experimentellen Eier erhalten. Die Methode ermöglicht die Bestimmung der Fruchtbarkeit mit mehr als 90% Genauigkeit am Tag "Null" (Tag der Eiablage) und des Geschlechtes der Küken mit mehr als 75% Genauigkeit am zwölften Bruttag.
Ein Nachteil besteht darin, dass mit dem Verfahren eine Geschlechtsbestimmung erst am 12. Tag durchgeführt werden kann.

In der Druckschrift US 6 029 080 B1 werden ein nichtinvasives Verfahren und eine Vorrichtung zur Geschlechtsbestimmung von Vogeleiern dargestellt, bei dem mittels magnetischer Kernresonanz (NMR) bestimmt wird, ob der lebende Embyo im Ei männliche Geschlechtsorgane oder weibliche Geschlechtsorgane aufweist.

Ein Nachteil besteht darin, dass die Ausbildung der Geschlechtsorgane erst im entwickelten Embryo nach wesentlichen mehreren Tagen erfolgt, wobei zumindest die Durchführung eines hohen finanziellen Aufwandes bedarf.

In der Druckschrift US 6 506 570 B1 wird zur in-ovo Geschlechtsbestimmung von Vogeleiern die An- oder Abwesenheit eines erhöhten geschlechtsspezifischen Hormonspiegels, vorzugsweise des Östrogenspiegels, in einer extraembryonalen Flüssigkeit, vorzugsweise der Allantoisflüssigkeit, bestimmt. Das Verfahren wird vorzugsweise auf Hühnereier angewendet und kann vor oder während der Umlagerung aus dem Vorbrüter in den Schlupfschrank durchgeführt werden.

In der Druckschrift DE 10 2012 023 947 A1 ist ein Verfahren zur Strukturaufklärung durch eine optisch undurchsichtige Barriere eines biologischen Untersuchungsobjektes hindurch beschrieben. Bei dem Untersuchungsobjekt wird eine innere Struktur mit unterschiedlichen dielektrischen Eigenschaften mittels elektromagnetischer Spektralanalyse aufgeklärt,
wobei das Untersuchungsobjekt unter einem Array von Pulssendern und Empfängern, welche in einer Ebene angeordnet und daten- sowie informationsleitend mit einem Computersystem verbunden sind, positioniert wird,
wobei durch die Pulssender elektromagnetischen Pulse im Spektralbereich 0,01 bis 1 THz auf das positionierte Untersuchungsobjekt ausgesendet werden,
wobei die vom Untersuchungsobjekt ausgehende Strahlung von den Empfängern aufgenommen und dem Computer über daten- sowie informationsleitende Verbindungen für ein bildgebendes Verfahren zugeleitet wird.
Da die THz-Strahlung sehr schwach ist, treten lange Messzeiten auf, die zur Durchführung einer schnellen Geschlechtsbestimmung hinderlich sind. Außerdem erfordert eine starke Absorption von begleitendem Wasserdampf im THz-Bereich eine extrem niedrige bzw. konstante Luftfeuchtigkeit in der Brüterei, was wiederum einen hohen technischen Zusatzaufwand bedeutet.

In der Druckschrift DE 20 2013 011 765 U1 ist eine spektralphotometrische Analyse der Federfarbe von Hühnerembryos beschrieben. Dabei wird elektromagnetische Energie mit einer Wellenlänge zwischen etwa 380nm und 740nm zur nicht invasiven Bestimmung der Geschlechts von Vogelembryos verwendet, wobei ein Vogelei der elektromagnetischen Energie ausgesetzt und die Menge an Absorption, Diffusion, Refraktion, Reflexion oder einer beliebeigen Kombination davon der elektromagnetischen Energie durch das Vogelei bestimmt wird. Es wird über das Vorliegen oder Fehlen von Farbpigment im Inneren des Vogeleies das Geschlecht des Vogelembryos wenigstens teilweise bestimmt.
Die spektralphotometrische Analyse der Federfarbe von Hühnerembryos kann nur auf braue Rassen bzw. auf Rassen mit Farbunterschied von weiblichen Küken oder männlichen Küken angewendet werden.

Im Folgenden wird eine Zusammenfassung der Nachteile der Verfahren aus den genannten Druckschriften angegeben:
1. die Geschlechtsbestimmung am Tage "Null" bedingt einen Zugang zur festgestellten Position der Keimscheibe, was nach bisherigen Erfahrungen eine Beeinträchtigung der Embryonalentwicklung und stark sinkende Schlupfraten mit sich bringen.
2. bei der späten Geschlechtbestimmung mit Inkubationstagen von 7 bis 21 Tagen spielen erstens tierschutzrechtliche Aspekte
   - wobei ab dem 7. Inkubationstag das Schmerzempfinden des Vogelembryos beginnt und
   - wobei ein spätes Abtöten weit entwickelter Vogelembryonen durchgeführt wird, weil mit steigender Inkubation der Ei-Inhalt aus dem Vogelembryo selbst besteht,
      und zweitens ökonomische Aspekte
   - wobei bei der späten Geschlechtsbestimmung die männlichen Eier länger im Brutschrank sind, was eine weitgehend schlechtere Auslastung der Brutschränke und somit höhere Stromkosten bedingen
      eine wesentliche Rolle.
3. Bei einer Geschlechtsbestimmung mit einer Entnahme von Probenmaterial können folgende Probleme auftreten:
   - die nach jeder Messung notwendige zusätzliche Reinigung und Desinfektion bzw. Ersatz von Geräten oder Geräteteilen (z.B. Kanülen) erhöht laufende Verbrauchskosten deutlich,
   - es ergibt sich eine erschwerte Automatisierbarkeit gegenüber kontaktlosen Verfahren und
   - das Infektionsrisiko wird stark erhöht, so dass sich eine Gefahr der reduzierten Schlupfraten ergeben kann.

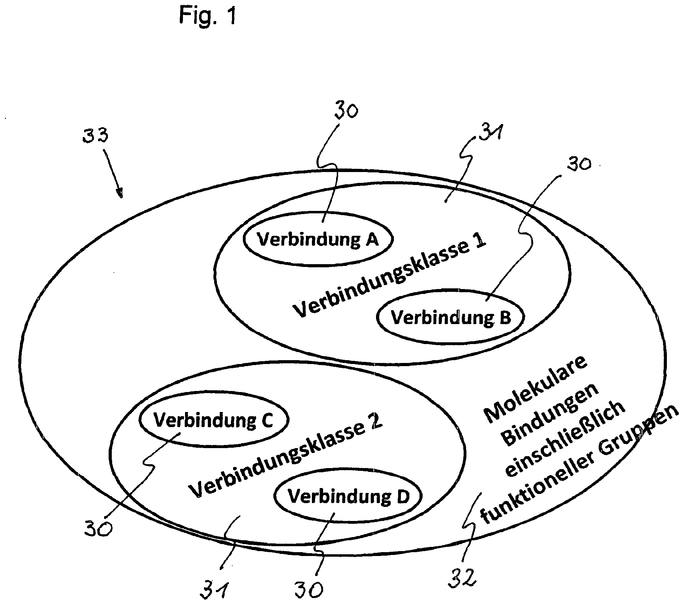

Alle in den oben genannten Druckschriften beschriebenen Verfahren und Vorrichtungen zur Geschlechtsbestimmung können in die schematisch in Fig. 1 dargestellten Auswertungs-Ebenen-Bereichsdarstellung 33 innerhalb der beiden inneren geschlossenen Gebiete 30, 31 der in Fig. 1 dargestellten drei Gebiete 30, 31, 32 eingeordnet werden. Die bekannten Verfahren umfassen in der Ebenen-Bereichsdarstellung 33 aber nicht den Restbereich 32, auf dem sich das vorliegende erfindungsgemäße Verfahren favorisiert.

In der Druckschrift: "Vibrational imaging and microspectroscopies based on coherent anti-Stokes Raman scattering microscopy", Andreas Volkmer, J. Phys. D: Appl. Phys. 38 (2005) R59-R81 doi:10.1088/0022-3727/3815/R01", einschließlich der darin enthaltenen Referenzen, wird die kohärente Anti-Stokes-Raman-Streuung Spektroskopie (Coherent Anti-Stokes Raman Scattering (CARS) spectroscopy) Theorie beschrieben, die zur nichtlinearen Ramanspektroskopie gehört. Mittels CARS-Spektroskopie werden die gleichen Molekülschwingungen wie bei der linearen Ramanspektroskopie untersucht.
Aber ein wesentlicher Unterschied zwischen der linearen und nichtlinearen Ramanspektroskopie besteht jedoch in der besonderen Anregungsart. Bei der CARS-Spektroskopie wird ein Multi-Photonen-Prozess zur Anregung der Molekülschwingungen genutzt und ein kohärentes Signal erzeugt. Im Ergebnis wird mittels CARS ein Signal erhalten, welches um Größenordnungen stärker als das der spontanen Ramanemission der linearen Ramanspektroskopie ist (Faktor 10⁵), was zu einer kürzeren Messzeit führt. Weiterhin ist das CARS-Signal blau verschoben und deshalb frei von Fluoreszenz.
Die Anregung erfolgt mittels ultraschneller NIR-Laser, sodass die Eindringtiefe vergleichbar mit der Eindringtiefe bei der NIR-Ramanspekrtroskopie ist und dadurch Lichtschäden minimiert sind.

Die Sensitivität wird nicht durch die Detektion der CARS-Photonen limitiert, sondern eher durch die Unterscheidung zwischen resonanten und nichtresonanten Anteilen des CARS-Signals. Verschiedene bekannte Verfahren existieren zum Separieren des resonanten Anteiles durch spezielle Konfigurationen zur Anregung/ Sammlung des Lichtes:
- Die polarisationssensitive Detektion,
- die zeitaufgelöste Detektion und
- die spektrale und räumliche Phasenkontrolle.
Alternativ kann das Ramanspektrum aus dem CARS-Spektrum durch eine modifizierte Kramers-Kronig-Transformation oder durch spezielle Rechenmethoden, basierend auf der Annahme maximaler Entropie, erhalten werden.

Verschiedene Anwendungen der Hochgeschwindigkeits-CARS-Spectroskopie sind in den Druckschriften:
"Unterscheidung biochemischer Komponentendiscrimination" A. Dogariu, Y. Huang, Y. Avitzour, R. K. Murawski, and M. O. Scully, und
"Sensitive femtosecond CARS discrimination between 2,6 Dipicolinic acid and 3,5 Dipicolinic acid," Opt. Lett., Vol. 31, No. 21, 3176 (2006);
Y. Huang, A. Soroka, K. Cohen, and A. Dogariu, und
"Backscattered Coherent Anti-Stokes Raman Scattering for At Range Detection of Dipicolinic Acid and Four-Wave Mixing Multiple Scattering," J. Mod. Optics Vol. 54, No. 16, 2473 (2007)), und
"Detektion von Bakterien" D. Pestov, X. Wang, G. O. Ariunbold, R. K. Murawski, V. A. Sautenkov, A. Dogariu, A. V. Sokolov, and M. O. Scully, und
"Single-shot Detection of Bacterial Endospores via Coherent Raman Spectroscopy," Proc. Nat. Acad. Sci. USA 105, 422 (2008); und
A. Dogariu, A. Goltsov, D. Pestov, A. V. Sokolov, and M. O. Scully, und "Real-time detection of bacterial spores using Coherent anti-Stokes Raman Spectroscopy," J. Appl. Phys. 103, 036103 (2008) und
die Echtzeitdetektion von Sprengstoffen in der Druckschrift
Coherent Anti-Stokes Raman Spectroscopy for detecting explosives in real-time, Arthur Dogariu and Alex Pidwerbetsky, Proc. of SPIE Vol. 8358, doi: 10.1117/12.919568) beschrieben.

Das genannte Ramanspektroskopische Verfahren kann ebenso an CARS-Spektren von Blut in Echtzeitmessungen demonstriert werden. Die CARS Schwingungsspektren der roten Blutzellen können in wenigen Picolitern und Millisekunden registriert werden, wie in folgenden Druckschriften A. Dogariu, A. Goltsov, and M. O. Scully, "Real-time monitoring of blood using coherent anti-Stokes Raman spectroscopy," J. Biomed. Opt. 13, 54004 (2008) und Rinia HA, Bonn M, Vartiainen EM, Schaffer CB, Müller M: Spectroscopic analysis of the oxygenation state of hemoglobin using coherent anti-stokes raman scattering. J. Biomed. Opt. 11(5):050502-050502-3 beschrieben ist.
Der Erfindung liegt deshalb die Aufgabe zugrunde, ein Verfahren und eine Vorrichtung zur Ramanspektroskopischen in-ovo Geschlechtsbestimmung von befruchteten und bebrüteten Vogeleiern anzugeben, die derart geeignet ausgebildet sind, dass das Geschlecht bereits in den Eiern schnell und zuverlässig eindeutig bestimmt werden kann, wobei sich der weibliche Embryo normal weiterentwickeln und es zum Schlupf des weiblichen Kükens kommen kann.
Die Aufgabe wird durch die Merkmale der Patentansprüche 1, 5 und 30 gelöst. Das Verfahren gemäß Anspruch 1 dient zur Ramanspektroskopischen in-ovo Geschlechtsbestimmung von befruchteten und bebrüteten Vogeleiern, wobei der sich ausbildende Embryo einschließlich der extraembryonalen Strukturen beweglich im Ei und zum Zeitpunkt eines Registrierens von Ramanstreustrahlung noch nicht an der Kalkschale fixiert ist,
wobei
folgende Schritte durchgeführt werden:
- Überwachung des Zeitverlaufs des Bebrütens bis zur Ausbildung mindestens eines erkennbaren Blutgefäßes,
- Schaffung eines Lochs in der Kalkschale zumindest im Nahbereich eines erkannten Blutgefäßes mittels einer Locherzeugungseinheit,
- Suchen von sich im Ei ausbildenden Blutgefäße mittels eines eine Strahlungseinrichtung zur koaxialen oder lateralen Beleuchtung mit Licht des sichtbaren Spektralbereichs und einen Detektor enthaltenden Visions-Systems,
- Positionieren zumindest eines Blutgefäßes in den Laserfokus einer Laserquelle entweder durch Bewegen des Eies oder eines Objektivs einer Vorrichtung zur Einbringung des Laserlichts und zur Erfassung der Ramanstreustrahlung, und Bestrahlen des Blutgefäßes mit Laserlicht,
- Registrieren der Ramanstreustrahlung des bestrahlten Blutgefäßes mittels der Vorrichtung zur Einbringung des Laserlichts und zur Erfassung der Ramanstreustrahlung, wobei während des Registrierens der Ramanstreustrahlung eine Bewegung des Blutgefäßes aus dem Fokus heraus durch Nachführung mittels des Visions-Systems vermieden wird,
- Auswertung der Ramanstreustrahlung und Bestimmung des Geschlechtes des Embryos in einer Auswerteeinheit,
- Anzeige des Geschlechtes des Embryos im Vogelei.
Das Einbringen eines Loches in die Kalkschale mittels einer Locherzeugungseinheit in Form eines Lasers oder mechanischer Perforation kann am nach oben gerichteten, spitzen Pol des Eies mit Durchmessern mit bis zu 18mm, vorzugsweise zwischen 8mm und 15mm durchgeführt werden.
Das Licht im sichtbaren Wellenlängenbereich kann weißes Licht sein, jedoch wird der Kontrast verbessert bei Einsatz von blauem und/oder grünem Licht aus einer Lichtquelle.
Während der Messung kann eine Bewegung des Blutgefäßes aus dem Fokus heraus durch Nachführung der Blutgefäße oder des zugehörigen Objektivs, ausgelöst durch das überwachende Vision-System, erfolgen. Das Verfahren gemäß Anspruch 5 dient zur Ramanspektroskopischen in-ovo Geschlechtsbestimmung von befruchteten und bebrüteten Vogeleiern, wobei die extraembryonalen Strukturen nicht mehr frei beweglich sind, sondern eine Fixierung der Chorioallantoismembran (CAM) an der Kalkschale beginnt,
wobei folgende Schritte durchgeführt werden:
- Überwachung des Zeitverlaufs des Bebrütens solange, bis eine Fixierung der Chorioallantoismembran (CAM) an der Kalkschale beginnt und bereits Blutgefäße vorhanden sind,
- Identifikation der Blutgefäßposition mittels Durchleuchtung mit Licht des sichtbaren Wellenlängenbereiches aus einer Lichtquelle eines eine Strahlungseinrichtung und einen Detektor enthaltenden Visions-Systems,
- Einbringen eines Loches in die Kalkschale mittels einer Locherzeugungseinheit in Form eines Lasers oder mittels mechanischer Perforation im Bereich der identifizierten Blutgefäße,
- Positionieren der Blutgefäße in den Laserfokus einer Laserquelle entweder durch Bewegen des Eies oder eines Objektivs einer Vorrichtung zur Einbringung von Laserlicht und zur Erfassung von Ramanstreustrahlung, und Bestrahlen der Blutgefäße mit Laserlicht,
- Registrieren der Ramanstreustrahlung zumindest eines bestrahlten Blutgefäßes, wobei die Ramanstreustrahlung des Blutes in den Kapillaren oder in größeren Gefäßen innerhalb oder unterhalb der Chorioallantoismembran (CAM) registriert wird,
- Auswertung der Ramanstreustrahlung und Bestimmung des Geschlechtes des Embryos in einer Auswerteeinheit,
- Anzeige des Geschlechtes des Embryos im Vogelei.
Das Licht des sichtbaren Wellenlängenbereiches kann weißes oder blaues oder grünes oder blaues und grünes, Kontrast erzeugendes Licht aus einer Lichtquelle sein.
Durch das Einbringen eines Loches in die Kalkschale mittels eines Lasers oder mittels mechanischer Perforation können Löcher mit Durchmessern mit bis zu 5mm, vorzugsweise zwischen 0,1 mm und 3mm ausgebildet werden,
Nach den jeweiligen Messabläufen und Verfahrensschritten erfolgt in der Auswerteeinheit eine Klassifizierung zur Erkennung des Geschlechtes des jeweils ausgemessenen bebrüteten Vogeleies vor der Anzeige des Geschlechtes.

Damit kann die Ramanstreustrahlung von Blut extraembryonaler Blutgefäße und/oder von Blut embryonaler Blutgefäße zur nicht invasiven in-ovo Geschlechtsbestimmung im bebrüteten Vogelei genutzt werden.
Folgende Parameter werden für die Ramanspektroskopie eingesetzt:
- Anregungswellenlängen des Laserlichts der Laserquelle für die Ramanspektroskopie: > 600 nm, z.B. HeNe-Laser 633 nm, Festkörperlaser (Nd basiert z.B. Nd:YAG Laser 1064nm, VIS NIR Diodenlaser z.B. 785 nm),
- Einkopplung des Laseranregungsstrahles direkt mit Spiegeln und/oder mit optischen Fasern.
- Ramanstreustrahlungsmessungen werden unter Nutzung von optischen, mit großer numerischer Apertur NA ausgebildeten Systemen wie Mikroskop-Objektiven oder einer Raman-Fasersonde durchgeführt,
- Direkte Auskopplung der gesammelten Ramanstreustrahlung mit Spiegeln zum Spektrometer oder für den Transport mit optischen Fasern,
- Anwendung von dispersiven Ramanspektrometern und Fourier Transform Raman Spektrometern.

Zur Bildung der genutzten Blutgefäße und der sich gebildeten Chorioallantoismembran (CAM) kann im jeweiligen Messablauf angegeben werden, dass sich die Blutgefäße ab dem zweiten Bebrütungstag bilden und bis zum dritten Bebrütungstag das embryonale Blut im Blutkreislauf zirkuliert. Das bedeutet, dass eine sofortige Geschlechtsbestimmung sehr frühzeitig etwa ab dem dritten Bruttag zweckmäßig ist.

Während des Positionierungsvorgangs des Vogeleies können permanent rückgeführte Ramanspektren aufgezeichnet und einer Auswertung zugeführt werden, wobei eine automatische Klassifizierung der Ramanstreustrahlung anhand des spektralen Fingerabdruckes des Blutes erfolgt.

Das Ei wird vorzugsweise pollastig gestellt und ein Loch kann im Bereich des nach oben gerichteten spitzen Pols bei einer Lochgröße von 10 mm und bei drei Bruttagen eingebracht werden. Etwa ab dem fünften Tag beginnt die Chorioallantoismembran an der äußeren Membran zu haften und es kann ein kleineres Loch in der Kalkschale bei Bruttagen mit länger als fünf Tagen, wobei das Loch auch horizontal möglich ist, eingebracht werden.

Das Loch der Kalkschale des Eies gewährleistet den optischen Zugang zu dem erkannten blutdurchflossenen Blutgefäß.

Der Laserstrahl wird auf das ausgewählte Blutgefäß fokussiert und die Nachführung mittels eines Objektivs gegebenenfalls automatisch durchgeführt.

Die eingebrachte Leistung der Laserquelle darf nicht zu einer lokalen und globalen Erwärmung des Eies über 40° Celsius führen.

Die Ramanstreustrahlung kann vorzugsweise mit einem Objektiv mit großer numerischer Apertur (NA > 0,3) registriert werden.

Die Ramanstreustrahlung kann mittels einer Raman-Fasersonde erfasst werden.

Die erfasste Ramanstreustrahlung kann über Faserleitungen dem Spektrometer zugeführt werden.

Für die Auswertung der Ramanbanden in der Auswerteeinheit wird vorzugsweise der Bereich von 500 cm⁻¹ bis 4000 cm⁻¹ (Raman-Verschiebung) genutzt.

Die geschlechtsspezifischen Merkmale sind in den Ramanbanden der Nukleinsäuren, Kohlenhydrate, Lipide und Proteine enthalten, wobei die Ramanbanden einer mathematischen Analyse zugeführt werden.

Für die mathematische Analyse können Verfahren der gestützten und nicht gestützten Klassifizierung eingesetzt werden, wobei die geschlechtsspezifischen Merkmale einer Merkmalstabelle abgelegt sind.

Die erfasste Ramanstreustrahlung kann in der Form korrigiert werden, dass Untergrundsignale durch Fluoreszenz oder andere Streuprozesse eliminiert werden und die Spektren in vorgegebener Form normiert werden.

Zur Geschlechtsbestimmung kann somit neben Blut auch die mit Kapillargefäßen durchzogene Chorioallantoismembran (CAM) herangezogen werden.

Die Geschlechtsbestimmung kann an jedem Tag zwischen dem Beginn der Bebrütung und dem Schlupf vorgenommen werden, vorzugsweise in Bezug auf die Ausbildung der Blutgefäße etwa am dritten Bruttag bis zum fünften Bruttag.

Anstelle der linearen Ramanspektroskopie kann die nichtlineare Ramanspektroskopie in Form der kohärenten Anti-Stokes-Raman-Streuung-Spektroskopie (CARS) genutzt werden, wobei das CARS-Spektrum vom Blut des Blutgefäßes unter Nutzung derselben Aufnahmekonfiguration wie für die spontane Ramanspektroskopie mit oder ohne Faseroptik aufgenommen wird.

Das CARS-Signal kann auch durch einen breitbandigen Femto-Sekundenlaser generiert werden, der als Pumplaser und als Stokeslaser dient.

Das CARS-Signal kann mittels zwei Laserquellen generiert werden, insbesondere mittels eines Breitbandlasers und eines Schmalbandlasers zur Nutzung und Auswertung einer multiplexen CARS-Spektroskopie.

Die CARS-Spektren können zur Klassifikation genutzt werden.

Der resonante Anteil des CARS-Spektrum [Im(Chi⁽³⁾)] kann vom nichtresonanten Anteil [Re(Chi⁽³⁾)] separiert und nur der resonante Anteil kann für die Klassifikation herangezogen werden.

Für die CARS-Spektroskopie kann eine gleiche Klassifikationsstrategie wie für die herkömmliche Ramanspektren durchgeführt werden.

Als Vogeleier kommen hauptsächlich Hühnereier in Betracht, deren Geschlecht bestimmt wird.

Die Vorrichtung zur Ramanspektroskopischen in-ovo Geschlechtsbestimmung von befruchteten und bebrüteten Vogeleiern gemäß Anspruch 30 nutzt die vorgenannten Verfahren und umfasst
zumindest
- eine Ei-Lagerungseinheit,
- eine Blutgefäß-Positions-Auswerteeinrichtung, die mit der Ei-Lagerungseinheit in Verbindung steht,
- eine Strahlungseinrichtung mit einer Lichtquelle zur Emission von Licht des sichtbaren Wellenlängenbereiches zur Erkennung mindestens eines Blutgefäßes, wobei die Strahlungseinrichtung zumindest einen Teil des Eies durchstrahlt,
- einen Detektor für das Licht zur Erkennung von Blutgefäßen, wobei der Detektor mit der Blutgefäß-Positions-Auswerteeinrichtung in Verbindung steht, wobei die Strahlungseinrichtung und der Detektor zumindest ein Visions-System bilden,
- eine Locherzeugungseinheit zur Schaffung eines Lochs in der Kalkschale im Nahbereich erkannter Blutgefäße,
- eine Laserquelle zur Emission von Laserlicht,
- eine optische Vorrichtung zur Einbringung des Laserlichts in das in der Kalkschale des Eies geschaffene Loch und zur Erfassung der Ramanstreustrahlung des Blutes des vom Laserlicht bestrahlten Blutgefäßes, wobei das Laserlicht auf mindestens ein Blutgefäß fokussiert gerichtet ist,
- ein Spektrometer zur Aufnahme der Ramanstreustrahlung des Blutes des vom Laserlicht bestrahlten Blutgefäßes über mindestens eine optische Faserleitung,
- eine Steuereinheit zur xyz-Positionierung der optischen Vorrichtung auf das in der Kalkschale des Eies geschaffene Loch, wobei die Steuereinheit mit der Blutgefäß-Positions-Auswerteeinrichtung in Verbindung steht und wobei die optische Vorrichtung mit der Laserquelle, dem Spektrometer und der Steuereinheit in Verbindung steht, und
- eine Geschlechtsbestimmungs-Auswerteeinheit, die mit dem Spektrometer und der Blutgefäß-Positions-Auswerteeinrichtung in Verbindung steht und aus der verarbeiteten erfassten Ramanstreustrahlung des Spektrometers das Geschlecht des bebrüteten Vogeleies angibt.

Der erfindungsgemäßen Vorrichtung kann als Visions-System eine Einrichtung zur Feststellung der Lage von Blutgefäßen innerhalb des Eies zugeordnet sein.

Die Einrichtung zur Feststellung der Lage eines Blutgefäßes ist über mindestens eine versorgungs- und signaltechnische Leitung mit der Steuereinheit verbunden.

Die Einrichtung zur Feststellung der Lage eines Blutgefäßes kann mit einer Höhenverstelleinrichtung und der Ei-Lagerungseinheit in Verbindung stehen.

Die Einrichtung zur Feststellung der Lage eines Blutgefäßes, die Höhenverstelleinrichtung und die Ei-Lagerungseinheit können mit der Blutgefäß-Positions-Auswerteeinrichtung mittels programmtechnischer Mittel zur Durchführung einer Koordinierung der Lage des Blutgefäßes verbunden sein.

Die optische Vorrichtung kann eine flexible optische Faser sein.

Die Strahlungseinrichtung kann vorzugsweise grünes Licht zur Erkennung zumindest eines Blutgefäßes emittieren.

Die Auswerteeinheit kann programmtechnische Mittel zur Geschlechtsbestimmungs-Auswertung der erfassten Ramanstreustrahlung und der erfassten CARS-Streustrahlung aufweisen.

Im Folgenden wird die Funktionsweise der erfindungsgemäßen Vorrichtung naher erläutert:
Über einem detektierten Blutgefäß wird ein Loch in die Kalkschale mittels einer Locherzeugungseinheit eingebracht. Auf das freigelegte Blutgefäß wird ein Laser fokussiert und vom gleichen Ort wird durch das Loch hindurch die Ramanstreustrahlung gesammelt und zurück zu einem Spektrometer zugeführt. Das alles geschieht unter Beobachtung, Überwachung und Kontrolle des mit sichtbarem/grünem Licht durchleuchteten Eies.
Mit einer ständigen Durchleuchtung des Eies kann eine zeitabhängige Bildung mindestens eines Blutgefäßes und der späteren Bildung auch der Chorioallantoismembran (CAM) überwacht werden, so dass der Beginn des Messablaufs für den Einsatz des erfindungsgemäßen Verfahrens auf die zeitliche Ausbildung des Blutgefäßes und später auch der Chorioallantoismembran (CAM) abgestimmt werden kann.

Es kann die Detektion der Blutgefäße insbesondere bei weißen Eiern mittels Einsatzes von weißem oder grünem Licht durchgeführt werden:
1. Variante: vom Bruttag 0 an bis maximal 4,5 Bruttage ist das Blutgefäßsystem einschließlich des Embryos noch beweglich. Nach der Öffnung des Eies erfolgt ein Absinken des Embryos und somit der Gefäße. Die Lage der Blutgefäße ist nach wenigen Minuten stabil, danach erfolgt eine Messung über ein größeres Loch vorzugsweise etwa 10 mm.
2. Variante: vom Bruttag 4,5 bis Bruttag 21 beginnt sich die Chorioallantoismembran (CAM) zu entwickeln und die Chorioallantoismembran beginnt fest an der Kalkschale zu haften. Dann kann die Messung bei einem kleineren Loch mit 0,1 mm bis 4 mm Durchmesser durchgeführt werden.

Die Ramanstreustrahlung wird spektral zerlegt aufgezeichnet und mit vorgegebenen mathematischen Verfahren bearbeitet und gemäß dem Geschlecht klassifiziert.

Zusammenfassend weist die Erfindung:
Verfahren und Vorrichtung zur Ramanspektroskopischen in-ovo Geschlechtsbestimmung von Vogeleiern, insbesondere von Hühnereiern,
folgende Schritte auf:
   In einem geschlossenen und bebrüteten Ei wird mittels grünem oder weißem oder blauem Licht die Lage von Blutgefäßen bestimmt und an vorgegebener Stelle wird ein Loch in die Kalkschale eingebracht, in dem mittels Laserstrahlung im roten oder nahen-infrarotem Spektralbereich die Ramanstreustrahlung von Blut oder Gewebe hinter der Kalkschale angeregt wird, sowie die Ramanstreustrahlung/das Ramanstreulicht registriert, einer spektralen und statistischen Auswertung zugeführt und das Geschlecht anhand charakteristischer Markerbanden bestimmt werden.

Für die Ramanspektroskopischen Messungen werden folgende Maßnahmen derart getroffen, dass
- das Ei bevorzugt steht und das Loch im Bereich des spitzen Pols eingebracht wird, wobei dies vor allem bei einer Lochgröße 10 mm bei drei Bruttagen erfolgt oder bei kleinerem Loch an Bruttagen > 5 Tage auch bei horizontaler Lage des Eies möglich ist,
- das Loch mittels eines Lasers oder mittels eines mechanischen Mittels in die Kalkschale eingebracht wird,
- das Loch der Kalkschale den optischen Zugang zu einem blutdurchflossenen Blutgefäß oder der Chorioallantoismembran (CAM) gewährleistet,
- der Laserstrahl auf das Blutgefäß fokussiert wird und gegebenenfalls automatisch nachgeführt wird (real-time tracking),
- die eingebrachte Leistung des Lasers nicht zu einer lokalen und globalen thermischen Schädigung führt,
- das Ramanstreulicht mit einem Objektiv mit großer numerischer Apertur (typische NA>0.3) gesammelt wird,
- das Ramanstreulicht mit einer Raman-Fasersonde aufgenommen wird,
- das aufgenommene Ramanstreulicht einem Spektrometer zugeführt wird,
- für die Auswertung der Ramanspektren vorzugsweise der Bereich von 500 bis 4000 cm-1 (Raman-Verschiebung) genutzt wird,
- die geschlechtsspezifischen Merkmale in den Ramanbanden verschiedener funktioneller Gruppen die z.B in Nukleinsäuren, Lipiden, Kohlenhydraten und Proteinen enthalten sind, die einer mathematischen Analyse zugeführt werden,
- für die mathematische Analyse Verfahren der gestützten und nicht gestützten Klassifizierung eingesetzt werden, wobei die geschlechtsspezifischen Merkmale in einer Merkmalstabelle abgelegt sind,
- die Ramanspektren in der Form korrigiert werden, dass Untergrundsignale durch Fluoreszenz oder andere Streuprozesse eliminiert werden und die Spektren in geeigneter Form normiert werden,
- zur Geschlechtsbestimmung neben dem Blut auch die von Blutkapillaren durchzogene CAM herangezogen werden kann,
- die Geschlechtsbestimmung an jedem Tag zwischen dem Beginn der Bebrütung und dem Schlupf vorgenommen werden kann.

In dem erfindungsgemäßen Verfahren wird Nahinfrarotlicht mit einer Wellenlänge von 785 nm (oder 1064 nm) zur Anregung der Ramanstreuung genutzt.

Die Vorteile der erfindungsgemäßen Geschlechtsbestimmung sind
- keine Beeinträchtigung des Schlupfes und der nachfolgenden Entwicklung des Kükens,
- Durchführung der Geschlechtsbestimmung mit hoher Genauigkeit, wobei vorzugsweise eine sichere Bestimmung der späteren geschlüpften Küken, insbesondere zu einem sehr frühen Zeitpunkt und eine kontaktlose Bestimmung ohne Probenentnahme erfolgen als es in den Verfahren und Vorrichtungen des Standes der Technik geschehen ist.

Mit dem erfindungsgemäßen Verfahren werden gemäß Fig. 1 keine Informationen über Verbindungen 30, sondern nur über molekulare Bindungen 32, unabhängig von den Verbindungen, erfasst, in denen die molekularen Bindungen 32 vorkommen. Zum Beispiel: Phosphatbindungen können in RNA, DNA, Phospholipiden usw. enthalten sein. Damit wird in der übergeordneten Ebene 32 gegenüber den die Realisierungs-Ebenen 30, 31 darstellenden, bereits veröffentlichten Druckschriften analysiert. In Relation zum Stand der Technik wird festgestellt, dass es generelle Unterschiede zu allen herkömmlichen chemisch-basierten Verfahren (spektroskopischen und nichtspektroskopischen) gibt:
Alle in den im Stand der Technik angegebenen Druckschriften genannten Verfahren erfassen die Anwesenheit/Quantität einzelner spezifischer Verbindungen 30 und Verbindungsklassen 31 (entweder DNA, Eiweiße, Zucker, Hormone).

In dem erfindungsgemäßen Verfahren und der zugehörigen Vorrichtung wird weder auf eine spezifische Verbindung 30 fokussiert noch werden absolute Mengen oder Mengenverhältnisse (Verbindungsklassen 31) im Vergleich zur Druckschrift WO 2014/021715 A2 berechnet.

Es werden gestützte Klassifikationsverfahren angewendet, um das gesamte Spektrum oder einen oder mehrere Spektralbereiche als männlich oder weiblich zu klassifizieren, basierend auf einem Trainingsset, das vorab aus bekannten Spektren oder Spektralbereichen erhalten werden kann.

Das jeweilige optische Element zur Strahlführung oder zur rückführenden Strahlführung kann eine flexible optische Faser sein.

Weiterbildungen und weitere Ausgestaltungen der Erfindung werden in weiteren Unteransprüchen angegeben.

Die Erfindung wird mittels eines Ausführungsbeispieles anhand zumindest einer Zeichnung näher erläutert.
Es zeigt:
- Fig. 1: eine Darstellung des Unterschiedes zwischen den Bestimmungs-Ebenen (Verbindung, Verbindungsklassen) des Standes der Technik und der Erfindungsebene (molekulare Bindungen einschließlich funktioneller Gruppen),
- Fig. 2: eine schematische Darstellung der erfindungsgemäßen Vorrichtung zur Bestimmung des Geschlechtes von befruchteten und bebrüteten Vogeleiern,
- Fig. 3: eine schematische Darstellung eines Teils der Vorrichtung zur Einbringung von Laserlicht in das Loch der Kalkschale, wobei
Fig. 3a den dem Ei zugewandten finalen Teil zur Bestrahlung des zumindest einen ermittelten Blutgefäßes und zur Erfassung der Ramanstreustrahlung von Seiten des bestrahlten Blutgefäßes aus und
Fig. 3b einen vergrößerten Ausschnitt mit Einblick in das Loch der kalkschale und den möglichen Messpunkten an mindestens einem Blutgefäß des beweglichen Embryos zeigen,
- Fig. 4: eine spektroskopische Darstellung der ausgewerteten Ramanstreustrahlungs-Intensitäten in einem vorgegebenen Wellenlängenbereich zur Erkennung des Unterschiedes zwischen weiblichen Vogeleiern und männlichen Vogeleiern, wobei die durchgezogene Linie das Mittelwertspektrum für den weiblichen Zustand und die gepunktete Linie das Mittelwertspektrum für den männlichen Zustand darstellen, und
- Fig. 5: ein Blockschema zur Erläuterung der Funktionsweise zur Klassifizierung der rückgeführten und detektierten Ramanspektren und damit zur Geschlechtsbestimmung des Vogeleiembryos.

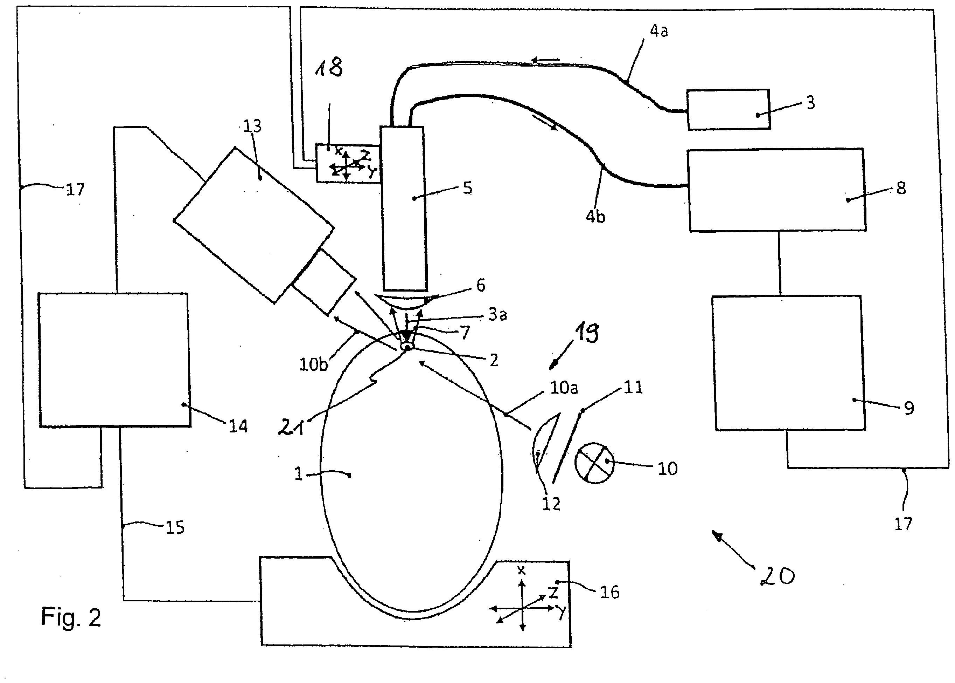

In Fig. 2 ist in einer schematischen Darstellung eine Vorrichtung 20 zur Ramanspektroskopischen in-ovo Geschlechtsbestimmung eines befruchteten und bebrüteten Vogeleies 1 gezeigt,
wobei die Vorrichtung 20 zumindest umfasst
- eine Ei-Lagerungseinheit 16, auf der das Ei 1 gelagert ist,
- eine Blutgefäß-Positions-Auswerteeinrichtung 14, die mit der Ei-Lagerungseinheit 16 in Verbindung steht,
- eine Strahlungseinrichtung 19 mit sichtbarem oder grünem Licht 10a einer Lichtquelle 10 zur Erkennung zumindest eines Blutgefäßes 21,
- einen Detektor 13 für sichtbares oder grünes Licht 10b zur Erkennung von Blutgefäßen 21, wobei der Detektor 13 mit der Blutgefäß-Positionier-Auswerteeinrichtung 14 in Verbindung steht,
- eine Locherzeugungseinheit für ein Loch 2 in der Kalkschale zumindest im Nahbereich des fixierten Blutgefäßes 21,
- eine Vorrichtung 5 zur Einbringung von Laserlicht 3a in das Ei 1,
   die zumindest in Verbindung steht mit
   - einer das Laserlicht 3a emittierende Laserquelle 3 und
   - einem Spektrometer 8 zur Aufnahme der Ramanstreustrahlung 7 und
   - einer Steuereinheit 18 zu xyz-Positionierung der Vorrichtung 5 auf das in das Ei 1 eingebrachte Loch 2,
   - eine Geschlechtsbestimmungs-Auswerteeinheit 9, die mit dem Spektrometer 8 und der Blutgefäß-Positionier-Auswerteeinrichtung 14 über der Steuereinheit 18 in Verbindung steht.

Der Vorrichtung 20 ist eine Einrichtung zur Feststellung der Lage der Blutgefäße 21 innerhalb des Eies 1 - ein Visions-System - in Verbindung mit der Strahlungseinrichtung 19 zugeordnet.
Die Einrichtung enthält des Weiteren einen Detektor 13 in Form einer Kamera.

Die Einrichtung zur Feststellung der Lage eines Blutgefäßes 21 ist über mindestens eine versorgungs- und signaltechnische Leitung 17 mit der Steuereinheit 18 in Form einer koordinativen Positioniereinheit verbunden.

Die Einrichtung zur Feststellung der Lage eines Blutgefäßes 21 steht mit einer Höhenverstelleinrichtung (nicht eingezeichnet) und der Ei-Lagerungseinheit 16 in Verbindung.

Die Einrichtung zur Feststellung der Lage des Blutgefäßes 21, die Höhenverstelleinrichtung, z.B. innerhalb der koordinativen Positioniereinheit 18 und die Ei-Lagerungseinheit 16 können in der Blutgefäß-Positions-Auswerteeinrichtung 14 mittels programmtechnischer Mittel zu einer Koordinierung und Feststellung der Lage des Blutgefäßes 21 verbunden sein.
Die Strahlungseinrichtung 19 zur Überwachung der Ausbildung von Blutgefäßen 21, 27 kann aus der Lichtquelle 10, einem Filter 11 und einer Linse 12 bestehen und kann auf den oberen Teil des Eies 1 gerichtet sein.
Die optische Vorrichtung 5 kann eine flexible optische Faser sein.
Dazu werden folgende Maßnahmen derart getroffen, dass
- das Ei 1 bevorzugt steht und das Loch 2 im Bereich des nach oben gerichteten spitzen Pols mit der Locherzeugungseinheit eingebracht wird, wobei Messungen vor allem bei einer Lochgröße 10 mm bei drei Bruttagen, bei kleinerem Loch bei Bruttagen > 5 Tage auch horizontal möglich sind,
- das Loch 2 in der Kalkschale mittels eines Lasers oder mittels eines mechanischen Mittels eingebracht wird,
- das Loch 2 der Kalkschale den optischen Zugang zu dem vordem erkannten blutdurchflossenen Blutgefäß 21 gewährleistet,
- der Laserstrahl 3a auf das Blutgefäß 21 innerhalb des Loches 2 fokussiert wird und gegebenenfalls automatisch nachgeführt wird,
- die eingebrachte Leistung der Laserquelle 3 nicht zu einer lokalen und globalen thermischen Schädigung des Eies 1 führt (Temperaturanstieg <1°C während der Messung),
- die Ramanstreustrahlung 7 mittels eines Objektivs 6 mit großer numerischer Apertur (NA>0,3) registriert wird,
- die Ramanstreustrahlung 7 mit einer Raman-Fasersonde 5 aufgenommen wird,
- die aufgenommene Ramanstreustrahlung 7 einem Spektrometer 8 über einer Faserleitung 4b zugeführt wird,
- für die Auswertung der Ramanbanden vorzugsweise der Bereich von 500cm⁻¹ bis 4000cm⁻¹ (Raman-Verschiebung) genutzt wird,
- die geschlechtsspezifischen Merkmale in den Ramanbanden einer mathematischen Analyse zugeführt werden,
- für die mathematische Analyse Verfahren der gestützten und nicht gestützten Klassifizierung eingesetzt werden, wobei die geschlechtsspezifischen Merkmale in einer Merkmalstabelle abgelegt sind,
- die Ramanspektren in der Form korrigiert werden, dass Untergrundsignale durch Fluoreszenz oder andere Streuprozesse eliminiert werden und die Spektren in vorgegebener Form normiert werden,
- zur erweiterten Geschlechtsbestimmung auch Membranen und Flüssigkeiten des Eies herangezogen werden können,
- die Geschlechtsbestimmung an jedem Tag zwischen dem Beginn der Bebrütung und dem Schlupf vorgenommen werden kann.

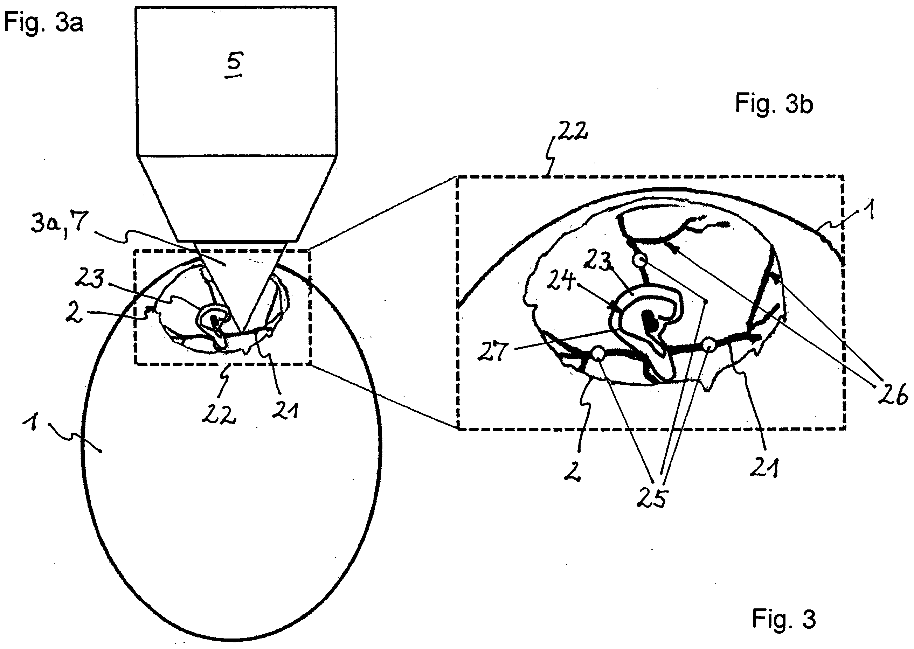

In Fig. 3 sind schematische Darstellungen eines Teils der Vorrichtung 5 zur Einbringung von Laserlicht 3a in das Loch 2 der Kalkschale gezeigt, wobei in Fig. 3a der dem Ei 1 zugewandte finale Teil zur Bestrahlung des zumindest einen ermittelten Blutgefäßes 21 und zur Erfassung der Ramanstreustrahlung 7 von Seiten des bestrahlten Blutgefäßes 21 aus und in Fig. 3b ein vergrößerter Ausschnitt mit Einblick in das Loch 2 der Kalkschale und den möglichen Messpunkten an mindestens dem Blutgefäß 21 des beweglichen Embryos 23 gezeigt sind.
In Fig. 3a wird von Seiten der Vorrichtung 5 ein auf das Blutgefäß 21 fokussiertes Laserlicht 3a gerichtet. Das Blutgefäß 21 befindet sich außerhalb des Embryos 23. Die entstehende Ramanstreustrahlung 7 wird über das Objektiv 6 von der Vorrichtung 5 erfasst und weitergeleitet. Der Embryo 23 besitzt ebenfalls Blutgefäße 27, die in Fig. 3b ebenfalls Ausgangsort der Messung einer Ramanstreustrahlung 7 sein können. In Fig. 3b ist ein vergrößerter Ausschnitt 22 des in das Ei 1 eingebrachten Loches 2 aus der Fig. 3a gezeigt. Unterhalb des Loches 2 der Kalkschale des Eies 1 befindet sich der Embryo 23 (Graufläche in dem Loch 2) mit seinen embryonalen Blutgefäßen 27 (geschwärzte Darstellungen) sowie mehrere extraembryonale Blutgefäße 21. Beide Blutgefäße 21 und 27 können Orte der Messungen und der Erfassung der Ramanstreustrahlung 7 sein, wobei die möglichen Messorte 24, 25, 26 je nach zeitlicher Ausbildung der Blutgefäße 21 und/oder 27 erkannt und mittels der programmtechnischen Mittel zumindest zum Vergleich mit gespeicherten Ramanspektren in den Steuer- und/oder Auswerteeinheiten 14, 9 festgelegt sein können.

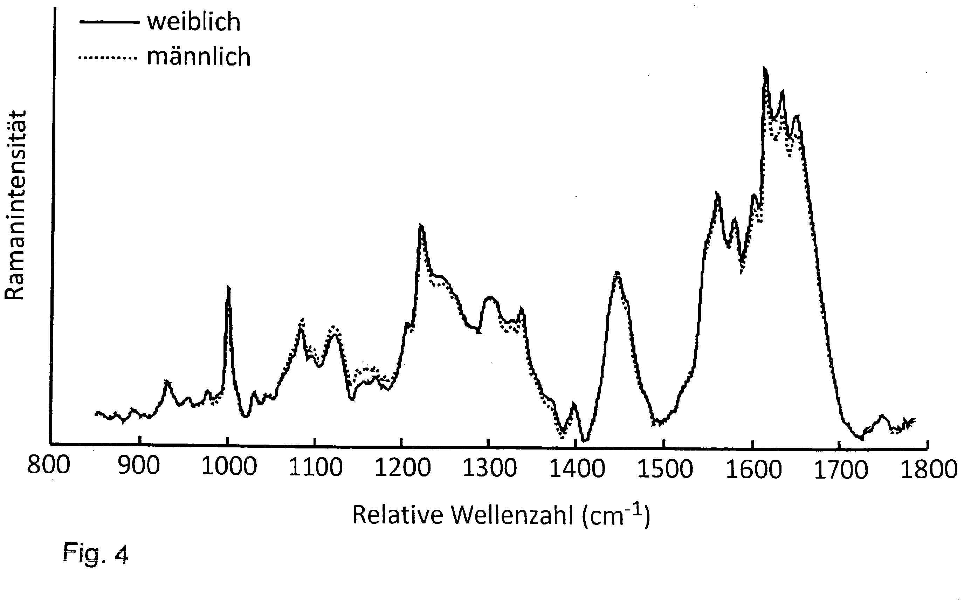

In Fig. 4 ist eine spektroskopische Darstellung der ausgewerteten Ramanstreustrahlungs-Intensitäten in einem vorgegebenen Wellenzahlbereich 800cm⁻¹ bis 1800cm⁻¹ zur Erkennung des Unterschiedes zwischen weiblichen Vogeleiern und männlichen Vogeleiern gezeigt, wobei die durchgezogene Linie das Mittelwertspektrum für den weiblichen Zustand und die gepunktete Linie das Mittelwertspektrum für den männlichen Zustand darstellen. Es gibt zwischen den Geschlechtskurven deutliche auf die jeweiligen Wellenzahlbereiche bezogenen Unterschiede bzw. Abstände zwischen den beiden Intensitäts-Wellenzahl-Kurven.

Die Untersuchungen und die Fig. 4 zeigen, dass z.B. drei bis vier spektrale Bereiche für die Geschlechtsbestimmung ausreichen können, z.B. kann für eine Geschlechtsbestimmung ein Vergleich der Intensitäten in den absoluten Beträgen durchgeführt werden: für das weibliche Geschlecht (durchgezogene Linie) ist der Intensitätsverlauf zwischen den vorgegebenen Wellenzahlen größer oder kleiner als der Intensitätsverlauf für das männliche Geschlecht (gepunktete Linie) zwischen den gleichen Wellenzahlen, so dass in einem der Wellenzahlbereiche wenigstens ein Intensitätsvergleich stattfinden kann.

Es kann auch eine Ausführung der Lichtquelle mit optischen Filtern der entsprechenden Wellenzahlen vorgesehen sein. Es kann anstelle eines teuren Spektrometers zumindest eine oder mehrere Laserdiode/n oder Lichtquelle/n mit einem wellenzahlzugeordneten Filter, insbesondere einem Interferenzfilter bezogen auf die vorgegebenen Wellenzahlen eingesetzt sein, wobei sich die Wellenzahl v aus dem Kehrwert der Wellenlänge A (in Mikrometer) multipliziert mit 10000 ergibt.

Bei der geschlechtsspezifischen Ramanstreuung des einfallenden IR- und/oder NIR-Lichts wird das Blut des Blutgefäßes 21 im Spektrometer 8 derart identifiziert, dass das Geschlecht des geprüften Vogeleis 1 bestimmt und angezeigt werden kann.

Die spektroskopische Auswertung erfolgt in der Auswerteeinheit 9 unter Einbeziehung mathematischer Klassifizierungsalgorithmen.

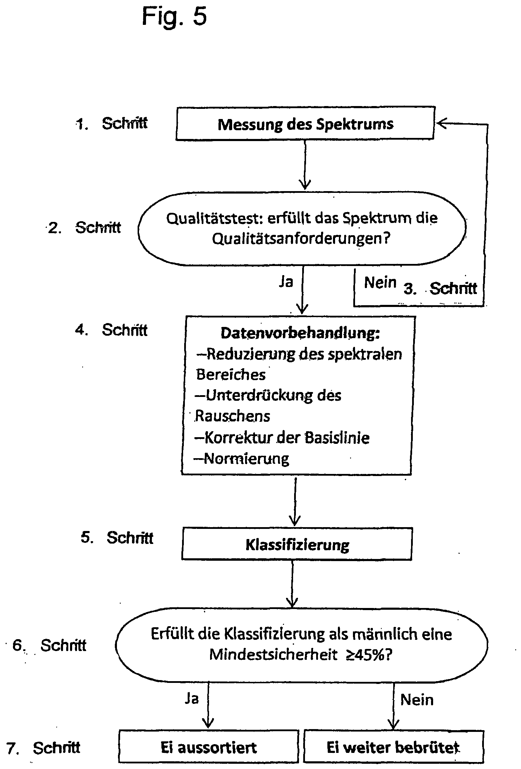

In Fig. 5 ist die Funktionsweise mit Einbeziehung der Klassifizierung der gemessenen Ramanspektren im Rahmen der Auswertung dargestellt.
Für die Durchführung der spektralen Klassifizierung und deren Ergebnisausgabe wird ein mehrstufiger Prozess angegeben:
1. Schritt der Messung der Ramanspektren des zu untersuchenden Eies 1,
2. Schritt des Qualitätstests der durchgeführten Messung zum Erkennen der Spektren und Eliminieren von unzureichend erkannten Spektren, mit denen keine Auswertung durchgeführt werden kann, wobei geprüft wird, ob die erfassten Spektren folgenden Anforderungen/Kriterien genügen:
   - das Verhältnis der integralen Intensitäten der CH₂/CH₃ - Streckschwingungen muss dem des Blutes entsprechen (abweichend vom umgebenden Gewebe oder Flüssigkeiten), bzw. das Verhältnis der Intensitäten von CH₂/CH₃ < 0.3.
   - das Signal-Rausch-Verhältnis (SNR) der Hämoglobin-Bande (750cm-1) hat mindestens eine Größe von 5:1
3. Schritt der Messwiederholung, falls die beiden Kriterien des vorhergehenden Schrittes nicht erreicht werden,
4. Schritt der Datenvorbehandlung mit
   - einer Reduzierung des spektralen Bereiches auf einen Wellenzahlbereich zwischen 500 cm⁻¹ und 4000 cm⁻¹,
   - einer Unterdrückung des Rauschens mittels Savitzky-Golayfilter,
   - einer Korrektur der Basislinie, z.B. mit einer polynomen Funktion und der Korrektur des Offsets,
   - einer Normierung der Spektren auf integrale Intensität durch Flächen- oder Vektornormierung.
5. Schritt der spektralen Klassifizierung mit
   der Anwendung einer unterstützten (engl. supervised) Klassifizierung. Als Klassifizierungsverfahren kann die SVM - Supporting Vector Machine, LDA - Lineare Diskriminanz Analyse, KNN-Nearest-Neighbour Klassification oder ANN-Artificial Neural Networks Verfahren - eingesetzt werden. Auch andere Verfahren, wie z.B. nichtlineare Verfahren/Methoden oder unterstützende Einrichtungen oder SIMCA, können eingesetzt werden. Die LDA klassifiziert mehrere spektrale Bereiche, also die Intensitätswerte dieser Bereiche.
6. gegebenenfalls Schritt der Verifizierung, wobei hierzu ein Referenzspektren-Set mit Referenzspektren "männlich" und Referenzspektren "weiblich" mit bekannter geschlechtlicher Zuordnung benötigt wird. Der Algorithmus vergleicht das Spektrum mit anderen Spektren der Geschlechtsklasse und überprüft die Ähnlichkeit des unbekannten Spektrums mit den bekannten gespeicherten Spektren.
7. Schritt zur Ausgabe der Ergebnisse der Bestimmung des jeweiligen Geschlechts der Vogeleier, wobei das Ei 1 aussortiert wird, wenn die Mindestsicherheit für "männlich" gleich oder unter 45% erreicht wird. Andernfalls liegt ein weibliches Ei 1 vor, das weiter bebrütet wird.

Das erfindungsgemäße Verfahren und die zugehörige Vorrichtung nutzen die Ramanspektren vorzugsweise von Blut extraembryonaler Blutgefäße zur nicht invasiven in-ovo Geschlechtsbestimmung im bebrüteten Vogelei 1.

Folgende Parameter für die Ramanspektroskopie werden eingesetzt:
- Anregungswellenlängen Laserlicht 3a: > 600 nm, z.B. HeNe Laser 633 nm, Festkörperlaser (Nd basiert z.B. Nd:YAG Laser 1064nm, VIS NIR Diodenlaser z.B. 785 nm),
- Einkopplung des Laseranregungsstrahles 3a direkt mit Spiegeln und/oder mit optischen Fasern,
- Ramanstreustrahlungsmessungen werden unter Nutzung der optischen Vorrichtungen 5 mit großer numerischer Apertur wie Mikroskop-Objektiven 6 oder einer Raman-Fasersonde 5 durchgeführt,
- Direkte Auskopplung der gesammelten Ramanstreustrahlung 7 mit Spiegeln zum Spektrometer oder Transport mit optischen Fasern,
- Anwendung von Spektrometern 8 in Form von dispersiven Ramanspektrometern und Fourier Transform Raman Spektrometern.

Zur Bildung der genutzten Blutgefäße 21 und der sich gebildeten Chorioallantoismembran (CAM) wird Folgendes angegeben:
Die Blutgefäße 21 bilden sich ab dem zweiten Bebrütungstag. Etwa am dritten Bebrütungstag zirkuliert das embryonale Blut im Blutkreislauf. In diesem Zeitbereich wird die Messung zur Geschlechtsbestimmung durchgeführt.

Folgende zwei erfindungsgemäße Verfahren werden somit genutzt:
Abhängig vom Bruttag existieren zwei Varianten zur Ramanstreustrahlungsmessung:
   1. Abschnitt der Zeitdauer der Bewegung des Embryos 23 und
   2. Abschnitt der Zeitdauer der Ausbildung der Chorioallantoismembran (CAM) und somit Fixierung des Embryos 23.

### Erste Variante:

Bis zum ca. vierten Bruttag ist der Embryo einschließlich der extraembryonalen Strukturen beweglich im Ei 1, d.h. sie sind nicht an der Kalkschale fixiert. Der Messablauf wird wie folgt durchgeführt:
- Überwachung bzw. Beobachtung des Zeitverlaufs des Bebrütens bis maximal zum vierten Bruttag,
- Einbringen eines Loches 2 in die Kalkschale mittels Laser oder mechanischer Perforation, vorzugsweise am spitzen Pol der Kalkschale des Eies 1 mit einem Laser, mit Durchmessern mit bis zu 18mm, vorzugsweise zwischen 8mm und 15mm,
- Suchen der Blutgefäße 21 mittels des Visions-Systems, vorzugsweise mit einer inline Visions-Kamera 13 und einer koaxialen oder lateralen Beleuchtung mit sichtbarem Licht 10a aus einer Lichtquelle 10. Das sichtbare Licht 10a kann weißes Licht sein, jedoch wird der Kontrast verbessert bei blauem und/oder grünem Licht. Die Visions-Kamera 13 nimmt das Licht 10b auf. Bringen der Blutgefäße 21 in den Laserfokus der Laserquelle 3, entweder durch Bewegen des Eies 1 oder des Objektives 6.
- Registrieren der Ramanstreustrahlung 7, wobei während der Messung eine mögliche Bewegung des Blutgefäßes 21 aus dem Fokus heraus durch real-time Tracking der Blutgefäße 21 durch die Überwachung mittels des Visions-Systems vermieden werden kann.
Vorzugsweise erfolgt eine Messung zum dritten Bruttag.

### Zweite Variante:

Ab dem fünften Bruttag sind die extraembryonalen Strukturen nicht mehr frei beweglich, d.h. die Fixierung der Chorioallantoismembran (CAM) an der Kalkschale 28 wird vollführt. Ab diesem Brutzeitpunkt gibt es eine Variante für den Messablauf, der wie folgt durchgeführt wird:
- Überwachung bzw. Beobachtung des Zeitverlaufs des Bebrütens ab dem fünften Bruttag,
- Identifikation der Position 25, 26 der Blutgefäße 21 oder der Position 24 der Blutgefäße 27 gemäß Fig. 3, 3a mittels Durchleuchtung mit weißem oder blauem oder grünem oder blauem und grünem Licht 10a,
- Einbringen eines Loches 2 in die Kalkschale im Bereich des identifizierten Blutgefäßes 21, 27 mittels eines Lasers oder mechanischer Perforation, vorzugsweise mit einem Laser, wobei die Löcher 2 mit Durchmessern bis zu 5mm, vorzugsweise zwischen 0.1 mm und 3mm ausgebildet werden,
- Bringen der Blutgefäße 21 in den Laserfokus entweder durch Bewegen des Eies 1 oder Bewegen des Objektives 6,
- Registrieren der Ramanstreustrahlung 7 in den Blutgefäßen 21, d.h. es erfolgt eine Messung des Blutes in den Kapillaren oder in größeren Blutgefäßen innerhalb oder unterhalb der Chorioallantoismembran (CAM).
Nach den jeweiligen Messabläufen erfolgt eine Klassifizierung zur Erkennung des Geschlechtes des jeweils ausgemessenen bebrüteten Vogeleies 1 in der Auswerteeinheit 9.
Anstelle der herkömmlichen linearen Ramanspektroskopie kann erfindungsgemäß die nichtlineare CARS-Spektroskopie zur Geschlechtsbestimmung genutzt werden.
Die kohärente Anti-Stokes-Raman-Streuung (Coherent Anti-Stokes Raman Scattering - CARS -) gehört zur nichtlinearen Ramanstreuung. Mittels CARS-Spektroskopie werden die gleichen Molekülschwingungen untersucht, jedoch besteht der Unterschied zur (linearen) Raman-Spektroskopie in der besonderen Anregungsart. Bei der CARS Spektroskopie wird ein Multi-Photonen Prozess zur Anregung der Molekülschwingungen genutzt und ein kohärentes Signal erzeugt. Im Ergebnis wird mittels CARS ein Signal erhalten, welches um Größenordnungen stärker als das der spontanen Ramanemission ist (Faktor 10⁵), was zu einer kürzeren Messzeit führt. Weiterhin ist das CARS Signal gegenüber der Anregungswellenlänge blau verschoben und deshalb frei von Fluoreszenz.
Die Anregung erfolgt mittels ultraschneller NIR-Laser, sodass die Eindringtiefe vergleichbar mit der Eindringtiefe bei der NIR-Ramanspektroskopie ist und die Lichtschäden minimiert sind.
Die Sensitivität wird nicht durch die Detektion der CARS Photonen limitiert, sondern eher durch die Unterscheidung zwischen resonanten und nichtresonanten Anteilen des CARS Signals. Verschiedene Verfahren existieren zum Separieren des resonanten Anteiles durch spezielle Konfigurationen zur Anregung/ Sammlung des Lichtes:
- Die polarisationssensitive Detektion,
- die zeitaufgelöste Detektion und
- die spektrale Phasenkontrolle und räumliche Phasenkontrolle.
Alternativ kann das Ramanspektrum aus dem CARS-Spektrum durch eine modifizierte Kramers-Kronig-Transformation oder durch spezielle Rechenmethoden, basierend auf der Annahme maximaler Entropie, erhalten werden.

Zur Durchführung des erfindungsgemäßen Verfahrens gemäß CARS erfolgt Folgendes:
- Das CARS-Spektrum vom Blut des Blutgefäßes 21, 27 wird erfasst unter Nutzung derselben Aufnahmekonfiguration wie für die spontane Ramanspektroskopie abhängig vom Brutzeitpunkt, mit oder ohne Faseroptik,
- Das CARS-Signal kann durch einen breitbandigen Femto-Sekundenlaser als Laserquelle 3 generiert werden, der als Pumplaser und Stokeslaser dient,
- Das CARS-Signal kann aber auch durch zwei Laserquellen 3 generiert werden, einen Breitbandlaser und einen Schmalbandlaser (multiplex CARS),
- Die CARS-Spektren werden zur Klassifikation genutzt.
- Der resonante Anteil des CARS-Spektrum [Im(Chi⁽³⁾)] wird vom nichtresonanten Anteil [Re(Chi⁽³⁾)] separiert und nur der resonante Anteil wird für die Klassifikation herangezogen.

Schließlich erfolgt eine gleiche Klassifikationsstrategie der CARS-Spektroskopie wie für die herkömmlichen linearen Ramanspektren gemäß dem Stand der Technik.

### Bezugszeichenliste

1 Ei
2 Loch in der Kalkschale
3 Laserquelle/Laserquellen
3a in das Loch der Kalkschale eingebrachtes Laserlicht
4a Laserlicht zum Ei führende optische Glasfaser
4b Ramanstreustrahlung zum Spektrometer/Detektor führende optische Glasfaser
5 Vorrichtung zur Einbringung des Laserlichtes in das Ei/ Fasersonde
6 Linse zur Fokussierung des Laserlichtes und zum Sammeln der Ramanstreustrahlung
7 Ramanstreustrahlung/Ramanstreulicht
8 Spektrometer mit Detektor
9 Auswerteeinheit
10 Lichtquelle
10a Licht, mit dem das Ei bestrahlt wird
10b transmittertes bzw. gestreutes sichtbares Licht
11 Filter grün
12 Linse
13 Kamera zur Detektion von Licht 10b
14 Blutgefäß-Positions-Auswerteeinrichtung
15 Steuerleitung
16 xyz-Positioniereinheit zur Ei-Lagesteuerung
17 Steuerleitungen
18 koordinative xyz-Positioniereinheit/Steuereinheit
19 Strahlungseinrichtung
20 erfindungsgemäße Vorrichtung
21 extraembryonales Blutgefäß
22 Ausschnitt aus Kalkschale
23 Embryo
24 Ort der Erfassung der Ramanstreustrahlung eines embryonalen Blutgefäßes 27
25 Orte der Erfassung der Ramanstreustrahlung eines extraembryonalen Blutgefäßes 21
26 Orte der Erfassung der Ramanstreustrahlung eines extraembryonalen Blutgefäßes 21
27 embryonales Blutgefäß
30 Gebiet der Verbindung
31 Gebiet der Verbindungsklassen
32 Gebiet der molekularen Bindungen
33 Erkennungs-Ebenen-Darstellung

## Patentansprüche

1. Verfahren zur Ramanspektroskopischen in-ovo Geschlechtsbestimmung von befruchteten und bebrüteten Vogeleiern (1), wobei der Embryo (23) einschließlich der extraembryonalen Strukturen beweglich im Ei (1) ist und zum Zeitpunkt eines Registrierens von Ramanstreustrahlung (7) noch nicht an der Kalkschale fixiert ist, mit folgenden Schritten:
- Überwachung des Zeitverlaufs des Bebrütens bis zur Ausbildung mindestens eines erkennbaren Blutgefäßes (21, 27),
- Schaffung eines Lochs (2) in der Kalkschale im Nahbereich eines erkannten Blutgefäßes (21, 27) mittels einer Locherzeugungseinheit,
- Suchen von sich im Ei (1) ausbildenden Blutgefäßen (21, 27) mittels eines eine Strahlungseinrichtung (19) zur koaxialen oder lateralen Beleuchtung mit Licht (10a) des sichtbaren Wellenlängenbereichs und einen Detektor (13) enthaltenden Visions-Systems (19, 13),
- Positionieren zumindest eines Blutgefäßes (21, 27) in den Laserfokus einer Laserquelle (3) entweder durch Bewegen des Eies (1) oder Bewegen eines Objektivs (6) einer Vorrichtung (5) zur Einbringung des Laserlichts (3a) und zur Erfassung der Ramanstreustrahlung (7), und Bestrahlen des Blutgefäßes mit Laserlicht (3a),
- Registrieren der Ramanstreustrahlung (7) des bestrahlten Blutgefäßes (21, 27) mittels der Vorrichtung (5) zur Einbringung des Laserlichts (3a) und zur Erfassung der Ramanstreustrahlung (7), wobei während des Registrierens der Ramanstreustrahlung (7) eine Bewegung des Blutgefäßes (21, 27) aus dem Fokus heraus durch Nachführung mittels des Visions-Systems (19, 13) vermieden wird,
- Auswertung der Ramanstreustrahlung (7) und Bestimmung des Geschlechtes des Embryos (23) in einer Auswerteeinheit (9), und
- Anzeige des Geschlechtes des Embryos (23) im Vogelei (1).

2. Verfahren nach Anspruch 1,
**dadurch gekennzeichnet,**
**dass** das Loch (2) am nach oben gerichteten, spitzen Pol des Eies (1) mit einem Durchmesser bis zu 18mm, vorzugsweise zwischen 8mm und 15mm mittels der Locherzeugungseinheit ausgebildet wird.

3. Verfahren nach Anspruch 1,
**dadurch gekennzeichnet,**
**dass** zur Überwachung des Zeitverlaufs des Bebrütens als Licht (10a) des sichtbaren Wellenlängenbereiches weißes oder blaues und/oder grünes Licht aus einer Lichtquelle (10) der Strahlungseinrichtung (19) des Vsions-Systems (19, 13) eingesetzt wird.

4. Verfahren nach einem der Ansprüche 1 bis 3, **dadurch gekennzeichnet,**
**dass** das Verfahren zur Ramanspektroskopischen in-ovo Geschlechtsbestimmung von befruchteten und bebrüteten Vogeleiern (1) ab dem dritten Bruttag durchgeführt wird.

5. Verfahren zur Ramanspektroskopischen in-ovo Geschlechtsbestimmung von befruchteten und bebrüteten Vogeleiern (1), wobei die extraembryonalen Strukturen nicht mehr frei beweglich sind und wobei eine Fixierung der Chorioallantoismembran an der Kalkschale beginnt, mit folgenden Schritten:
- Überwachung des Zeitverlaufs des Bebrütens bis eine Fixierung der Chorioallantoismembran an der Kalkschale beginnt und bereits Blutgefäße (21, 27) vorhanden sind,
- Identifikation der Blutgefäßposition (24, 25, 26) mittels Durchleuchtung mit Licht (10a) des sichtbaren Wellenlängenbereiches aus einer Lichtquelle (10) eines eine Strahlungseinrichtung (19) und einen Detektor (13) enthaltenden Visions-Systems (19, 13),
- Einbringen eines Loches (2) in die Kalkschale mittels einer Locherzeugungseinheit in Form eines Lasers oder mittels mechanischer Perforation im Bereich der identifizierten Blutgefäße (21, 27),
- Positionieren der Blutgefäße (21, 27) in den Laserfokus einer Laserquelle (3) entweder durch Bewegen des Eies (1) oder eines Objektivs (6) einer Vorrichtung (5) zur Einbringung von Laserlicht (3a) und zur Erfassung von Ramanstreustrahlung (7), und Bestrahlen der Blutgefäße mit Laserlicht (3a),
- Registrieren der Ramanstreustrahlung (7) zumindest eines bestrahlten Blutgefäßes (21, 27), wobei die Ramanstreustrahlung des Blutes in den Kapillaren oder in größeren Blutgefäßen innerhalb oder unterhalb der Chorioallantoismembran registriert wird,
- Auswertung der Ramanstreustrahlung (7) und Bestimmung des Geschlechtes des Embryos (23) in einer Auswerteeinheit (9), und
- Anzeige des Geschlechtes des Embryos (23) im Vogelei (1).

6. Verfahren nach Anspruch 5,
**dadurch gekennzeichnet,**
**dass** ein Loch (2) mit einem Durchmesser bis zu 5 mm, vorzugsweise zwischen 0,1 mm und 3 mm mittels der Locherzeugungseinheit ausgebildet wird.

7. Verfahren nach Anspruch 5,
**dadurch gekennzeichnet,**
**dass** zur Überwachung des Zeitverlaufs des Bebrütens als Licht (10a) des sichtbaren Wellenlängenbereiches weißes oder blaues und/oder grünes Licht aus der Lichtquelle (10) der Strahlungseinrichtung (19) des Visions-Systems (19, 13) eingesetzt wird.

8. Verfahren nach einem der Ansprüche 5 bis 7, **dadurch gekennzeichnet,**
**dass** das Verfahren zur Ramanspektroskopischen in-ovo Geschlechtsbestimmung von befruchteten und bebrüteten Vogeleiern (1) ab dem fünften Bruttag durchgeführt wird.

9. Verfahren nach einem der Ansprüche 1 bis 8, **dadurch gekennzeichnet,**
**dass** nach den jeweiligen Messabläufen eine Klassifizierung zur Erkennung des Geschlechtes des jeweils ausgemessenen bebrüteten Vogeleies (1) vor der Anzeige des Geschlechtes erfolgt.

10. Verfahren nach Anspruch 1 oder 5,
**dadurch gekennzeichnet,**
**dass** die Ramanstreustrahlung (7) von Blut extraembryonaler Blutgefäße (21) und/oder von Blut embryonaler Blutgefäße (27) zur nicht invasiven in-ovo Geschlechtsbestimmung im bebrüteten Vogelei (1) genutzt wird.

11. Verfahren nach einem der Ansprüche 1 bis 10, **dadurch gekennzeichnet,**
**dass** folgende Parameter für die Ramanspektroskopie eingesetzt werden:
- Anregungswellenlängen des Laserlichts (3a) der Laserquelle (3): > 600 nm, beispielsweise 633 nm eines HeNe Lasers, 1064 nm eines Nd:YAG Festkörperlasers oder 785 nm eines VIS NIR Diodenlasers,
- Einkopplung des Laserlichts (3a) direkt mit Spiegeln und/oder mit optischen Fasern (4a),
- Durchführung von Ramanstreustrahlungsmessungen unter Nutzung von optischen Vorrichtungen (5) mit großer numerischer Apertur wie Mikroskop-Objektiven (6) oder einer Raman-Fasersonde,
- Direkte Auskopplung der gesammelten Ramanstreustrahlung (7) mit Spiegeln zu einem Spektrometer (8) oder mittels Transports mit optischen Fasern (4b),
- Einsatz des Spektrometers (8) in Form von dispersivem Ramanspektrometer und Fourier Transform Ramanspektrometer.

12. Verfahren nach einem der Ansprüche 1 bis 11, **dadurch gekennzeichnet,**
**dass** während des Positionierungsvorgangs des Vogeleis (1) permanent rückgeführte Ramanspektren aufgezeichnet und einer Auswertung zugeführt werden, wobei eine automatische Klassifizierung der Ramanstreustrahlung (7) anhand des spektralen Fingerabdruckes des Blutes erfolgt.

13. Verfahren nach einem der Ansprüche 1 bis 12, **dadurch gekennzeichnet,**
**dass** das Ei (1) pollastig gestellt wird und das Loch (2) im Bereich des nach oben gerichteten spitzen Pols bei einer Lochgröße von 10 mm bei drei Bruttagen oder bei kleinerem Loch, auch in möglicher Horizontallage, an Bruttagen, die länger als fünf Tage dauern, eingebracht wird.

14. Verfahren nach einem der Ansprüche 1 bis 13, **dadurch gekennzeichnet,**
**dass** das Loch (2) der Kalkschale des Eies (1) einen optischen Zugang zu einem blutdurchflossenen Blutgefäß (21, 27) gewährleistet.

15. Verfahren nach einem der Ansprüche 1 bis 14, **dadurch gekennzeichnet,**
**dass** das Laserlicht (3a) auf das Blutgefäß (21, 27) automatisch fokussiert wird.

16. Verfahren nach einem der Ansprüche 1 bis 15, **dadurch gekennzeichnet,**
**dass** die eingebrachte Leistung der Laserquelle (3) nicht zu einer lokalen und globalen Erwärmung des Eies (1) über 40° Celsius führt.

17. Verfahren nach einem der Ansprüche 1 bis 16, **dadurch gekennzeichnet,**
**dass** die Ramanstreustrahlung (7) mit einem Objektiv (6) mit großer numerischer Apertur von NA > 0,3 registriert wird.

18. Verfahren nach einem der Ansprüche 1 bis 17, **dadurch gekennzeichnet,**
**dass** die Ramanstreustrahlung (7) mit einer Raman-Fasersonde aufgenommen wird.

19. Verfahren nach einem der Ansprüche 11 oder 12 bis 18, wenn abhängig von Anspruch 11, **dadurch gekennzeichnet,**
**dass** die erfasste Ramanstreustrahlung (7) über Faserleitungen (4b) dem Spektrometer (8) zugeführt wird.

20. Verfahren nach einem der Ansprüche 1 bis 19, **dadurch gekennzeichnet,**
**dass** für die Auswertung der Ramanstreustrahlung in der Auswerteeinheit (9) der Bereich von 500 cm⁻¹ bis 4000 cm⁻¹ der Raman-Verschiebung genutzt wird.

21. Verfahren nach einem der Ansprüche 1 bis 20, **dadurch gekennzeichnet,**
**dass** die geschlechtsspezifischen Merkmale in den Ramanbanden der Nukleinsäuren, Kohlenhydrate, Lipide und Proteine enthalten sind, die einer mathematischen Analyse zugeführt werden.

22. Verfahren nach Anspruch 21,
**dadurch gekennzeichnet,**
**dass** für die mathematische Analyse Verfahren der gestützten und nicht gestützten Klassifizierung eingesetzt werden, wobei die geschlechtsspezifischen Merkmale in einer Merkmalstabelle einer Auswerteeinheit (9) abgelegt sind.

23. Verfahren nach einem der Ansprüche 1 bis 22, **dadurch gekennzeichnet,**
**dass** die erfasste Ramanstreustrahlung (7) in der Form korrigiert wird, dass störende Untergrundsignale von Streuprozessen eliminiert werden und die Spektren in vorgegebener Form normiert werden.

24. Verfahren nach einem der Ansprüche 1 bis 23, **dadurch gekennzeichnet,**
**dass** als Ramanspektroskopie die nichtlineare kohärente Anti-Stokes-Raman-Streuung-Spektroskopie CARS genutzt wird, wobei die CARS-Streustrahlung vom Blut des Blutgefäßes (21, 27) unter Nutzung derselben Vorrichtung (20) wie für die spontane lineare Ramanspektroskopie mit oder ohne Faseroptik (5) aufgenommen wird.

25. Verfahren nach Anspruch 24,
**dadurch gekennzeichnet,**
**dass** das CARS-Signal durch einen breitbandigen Femto-Sekundenlaser als Laserquelle (3) generiert wird, der als Pumplaser und als Stokeslaser dient.

26. Verfahren nach Anspruch 25,
**dadurch gekennzeichnet,**
**dass** das CARS-Signal durch zwei Laser generiert wird, insbesondere durch einen Breitbandlaser und einen Schmalbandlaser als Laserquellen (3) zur Nutzung und Auswertung einer multiplexen CARS-Spektroskopie.

27. Verfahren nach einem der Ansprüche 24 bis 26, **dadurch gekennzeichnet,**
**dass** die CARS-Spektren für eine Klassifikation zum Erkennen des Geschlechts genutzt werden.

28. Verfahren nach Anspruch 27, **dadurch gekennzeichnet,**
**dass** der resonante Anteil des CARS-Spektrum [Im(Chi⁽³⁾)] vom nichtresonanten Anteil [Re(Chi⁽³⁾)] separiert und nur der resonante Anteil für die Klassifikation herangezogen werden.

29. Verfahren nach einem der Ansprüche 24 bis 28, **dadurch gekennzeichnet,**
**dass** für die CARS-Spektroskopie eine gleiche Klassifikationsstrategie zum Erkennen des Geschlechts wie für die linearen Ramanspektren durchgeführt wird.

30. Vorrichtung zur Ramanspektroskopischen in-ovo Geschlechtsbestimmung von befruchteten und bebrüteten Vogeleiern (1), unter Verwendung der Verfahren nach einem der Ansprüche 1 bis 29, zumindest umfassend
- eine Ei-Lagerungseinheit (16),
- eine Blutgefäß-Positions-Auswerteeinrichtung (14), die mit der Ei-Lagerungseinheit (16) in Verbindung steht,
- eine Strahlungseinrichtung (19) mit einer Lichtquelle (10) zur Emission von Licht (10a) des sichtbaren Wellenlängenbereiches zur Erkennung mindestens eines Blutgefäßes (21, 27), wobei die Strahlungseinrichtung (19) zumindest einen Teil des Eies (1) durchstrahlt,
- einen Detektor (13) für das Licht (10b) zur Erkennung von Blutgefäßen (21, 27), wobei der Detektor (13) mit der Blutgefäß-Positions-Auswerteeinrichtung (14) in Verbindung steht, wobei die Strahlungseinrichtung (19) und der Detektor (13) zumindest ein Visions-System (19, 13) bilden,
- eine Locherzeugungseinheit zur Schaffung eines Lochs (2) in der Kalkschale im Nahbereich erkannter Blutgefäße (21, 27),
- eine Laserquelle (3) zur Emission von Laserlicht (3a),
- eine optische Vorrichtung (5) zur Einbringung des Laserlichts (3a) in das in der Kalkschale des Eies (1) geschaffene Loch (2) und zur Erfassung der Ramanstreustrahlung (7) des Blutes des vom Laserlicht (3a) bestrahlten Blutgefäßes (21, 27), wobei das Laserlicht (3a) auf mindestens ein Blutgefäß (21, 27) fokussiert gerichtet ist,
- ein Spektrometer (8) zur Aufnahme der Ramanstreustrahlung (7) des Blutes des vom Laserlicht (3a) bestrahlten Blutgefäßes (21, 27) über mindestens eine optische Faserleitung (4b),
- eine Steuereinheit (18) zur xyz-Positionierung der optischen Vorrichtung (5) auf das in der Kalkschale des Eies (1) geschaffene Loch (2), wobei die Steuereinheit mit der Blutgefäß-Positions-Auswerteeinrichtung in Verbindung steht und wobei die optische Vorrichtung (5) mit der Laserquelle (3), dem Spektrometer (8) und der Steuereinheit (18) in Verbindung steht, und
- eine Geschlechtsbestimmungs-Auswerteeinheit (9), die mit dem Spektrome-ter (8) und der Blutgefäß-Positions-Auswerteeinrichtung (14) in Verbindung steht und aus der verarbeiteten erfassten Ramanstreustrahlung (7) des Spektrometers (8) das Geschlecht des bebrüteten Vogeleies (1) angibt.

31. Vorrichtung nach Anspruch 30,
**dadurch gekennzeichnet,**
**dass** die Locherzeugungseinheit in Form eines Lasers oder einer Einrichtung zur mechanischen Perforation mit der Blutgefäß-Positions-Auswerteeinrichtung (14) über eine Leitung in Verbindung steht.

32. Vorrichtung nach Anspruch 30,
**dadurch gekennzeichnet,**
**dass** das Visions-System (19, 13) eine Einrichtung zur Feststellung der Lage der Blutgefäße (21,27) mit dem Detektor (13) ist.

33. Vorrichtung nach Anspruch 32,
**dadurch gekennzeichnet,**
**dass** die Einrichtung zur Feststellung der Lage eines Blutgefäßes (21, 27) über mindestens eine versorgungs- und signaltechnische Leitung (17) mit der Steuereinheit (18) verbunden ist.

34. Vorrichtung nach einem der Ansprüche 32 oder 33, **dadurch gekennzeichnet,**
**dass** die Steuereinheit (18) in Form einer koordinativen Positioniereinheit ausgestaltet ist und dass die Einrichtung zur Feststellung der Lage eines Blutgefäßes (21, 27) mit einer innerhalb der koordinativen Positioniereinheit (18) befindlichen Höhenverstelleinrichtung und der Ei-Lagerungseinheit (16) in Verbindung steht.

35. Vorrichtung nach Anspruch 34,
**dadurch gekennzeichnet,**
**dass** die Einrichtung zur Feststellung der Lage des Blutgefäßes (21, 27), die Höhenverstelleinrichtung und die Ei-Lagerungseinheit (16) in der Blutgefäß-Positions-Auswerteeinrichtung (14) mittels programmtechnischer Mittel zur Feststellung der Lage des Blutgefäßes (21, 27) verbunden sind.

36. Vorrichtung nach einem der Ansprüche 30 bis 35, **dadurch gekennzeichnet,**
**dass** die optische Vorrichtung (5) eine flexible optische Faser ist.

37. Vorrichtung nach einem der Ansprüche 30 bis 36, **dadurch gekennzeichnet,**
**dass** die Strahlungseinrichtung (19) Licht (10a) des sichtbaren Wellenlängenbereiches, zumindest grünes Licht (10a) zur Erkennung mindestens eines Blutgefäßes (21, 27) emittiert.

38. Vorrichtung nach einem der Ansprüche 30 bis 37, **dadurch gekennzeichnet,**
**dass** die Auswerteeinheit (9) programmtechnische Mittel zur Geschlechtsbestimmungs-Auswertung der erfassten Ramanstreustrahlung (7) aufweist.

39. Vorrichtung nach einem der Ansprüche 30 bis 38, **dadurch gekennzeichnet,**
**dass** die Vorrichtung (5) zur Einbringung des Laserlichts (3a) und der Erfassung der Ramanstreustrahlung (7) mit der Steuereinheit (18) in Verbindung steht, die das Laserlicht (3a) bei beweglichem Embryo (23) auf das ermittelte erfassbare Blutgefäß (21, 27) fokussieren hilft.

## Claims

1. Method for determining the sex of fertilized and incubated bird eggs (1) in ovo by way of Raman spectroscopy, wherein the embryo (23) including the extraembryonic structures is movable in the egg (1) and not yet attached to the eggshell at the time of registering Raman scattering radiation (7), including the following steps:
- monitoring the passage of time of the incubation up to the formation of at least one identifiable blood vessel (21, 27),
- creating a hole (2) in the eggshell in the vicinity of an identified blood vessel (21, 27) by means of a hole-producing unit,
- searching for blood vessels (21, 27) forming in the egg (1) by means of a vision system (19, 13) containing a radiation device (19) for coaxial or lateral illumination with light (10a) in the visible wavelength range and a detector (13),
- positioning at least one blood vessel (21, 27) in the laser focus of a laser source (3), either by moving the egg (1) or by moving an objective lens (6) of an apparatus (5) for introducing the laser light (3a) and for capturing the Raman scattered radiation (7), and irradiating the blood vessel with laser light (3a),
- registering the Raman scattered radiation (7) of the irradiated blood vessel (21, 27) by means of the apparatus (5) for introducing the laser light (3a) and for capturing the Raman scattered radiation (7), wherein a movement of the blood vessel (21, 27) out of the focus during the registration of the Raman scattered radiation (7) is avoided by tracking by means of the vision system (19, 13),
- evaluating the Raman scattered radiation (7) and determining the sex of the embryo (23) in an evaluation unit (9), and
- displaying the sex of the embryo (23) in the bird egg (1).

2. Method according to Claim 1,
**characterized**
**in that** the hole (2) is formed at the upwardly directed, peaked pole of the egg (1) with a diameter of up to 18 mm, preferably of between 8 mm and 15 mm, by means of the hole-producing unit.

3. Method according to Claim 1,
**characterized**
**in that** white or blue and/or green light from a light source (10) of the radiation device (19) of the vision system (19, 13) is used as light (10a) of the visible wavelength range for monitoring the passage of time of the incubation.

4. Method according to any one of Claims 1 to 3,
**characterized**
**in that** the method for determining the sex of fertilized and incubated bird eggs (1) in ovo by way of Raman spectroscopy is carried out from the third day of incubation onward.

5. Method for determining the sex of fertilized and incubated bird eggs (1) in ovo by way of Raman spectroscopy, wherein the extraembryonic structures are no longer freely movable and wherein the chorioallantoic membrane starts to be affixed to the eggshell, including the following steps:
- monitoring the passage of time of the incubation until chorioallantoic membrane starts to be affixed to the eggshell and blood vessels (21, 27) are already present,
- identifying the blood vessel positions (24, 25, 26) by transillumination using light (10a) from the visible wavelength range from a light source (10) of a vision system (19, 13) containing a radiation device (19) and a detector (13),
- introducing a hole (2) into the eggshell by means of a hole-producing unit in the form of a laser or by means of mechanical perforation in the region of the identified blood vessels (21, 27),
- positioning the blood vessels (21, 27) into the laser focus of a laser source (3) either by moving the egg (1) or an objective lens (6) of an apparatus (5) for introducing laser light (3a) and for capturing Raman scattered radiation (7), and irradiating the blood vessels with laser light (3a),
- registering the Raman scattered radiation (7) of at least one irradiated blood vessel (21, 27), wherein the Raman scattered radiation of the blood is registered in the capillaries or in larger blood vessels within or below the chorioallantoic membrane,
- evaluating the Raman scattered radiation (7) and determining the sex of the embryo (23) in an evaluation unit (9), and
- displaying the sex of the embryo (23) in the bird egg (1).

6. Method according to Claim 5,
**characterized**
**in that** a hole (2) with a diameter up to 5 mm, preferably between 0.1 mm and 3 mm, is formed by means of a hole-producing unit.

7. Method according to Claim 5,
**characterized**
**in that** white or blue and/or green light from the light source (10) of the radiation device (19) of the vision system (19, 13) is used as light (10a) of the visible wavelength range for monitoring the passage of time of the incubation.

8. Method according to any one of Claims 5 to 7,
**characterized**
**in that** the method for determining the sex of fertilized and incubated bird eggs (1) in ovo by way of Raman spectroscopy is carried out from the fifth day of incubation onward.

9. Method according to any one of Claims 1 to 8,
**characterized**
**in that**, after the respective measurement procedures, there is a classification for identifying the sex of the respectively measured incubated bird egg (1) before displaying the sex.

10. Method according to Claim 1 or 5,
**characterized**
**in that** the Raman scattered radiation (7) of blood of extraembryonic blood vessels (21) and/or of blood of embryonic blood vessels (27) is used for determining the sex in the incubated bird egg (1) in a non-invasive fashion.

11. Method according to any one of Claims 1 to 10,
**characterized**
**in that** the following parameters are used for Raman spectroscopy:
- excitation wavelength of the laser light (3a) of the laser source (3): > 600 nm, for example 633 nm of a HeNe laser, 1064 nm of an Nd:YAG solid-state laser or 785 nm of a VIS NIR diode laser,
- coupling-in the laser light (3a) directly with mirrors and/or with optical fibres (4a),
- carrying out Raman scattered radiation measurements using optical apparatuses (5) with a large numerical aperture such as microscope objectives (6) or a Raman fibre probe,
- directly decoupling the collected Raman scattered radiation (7) by means of mirrors to a spectrometer (8) or by means of a transport using optical fibres (4b),
- using the spectrometer (8) in the form of a dispersive Raman spectrometer and Fourier Transform Raman spectrometer.

12. Method according to any one of Claims 1 to 11,
**characterized**
**in that** Raman spectra that are permanently fed back during the positioning process of the bird egg (1) are recorded and fed to an evaluation, wherein there is an automatic classification of the Raman scattered radiation (7) on the basis of the spectral fingerprint of the blood.

13. Method according to any one of Claims 1 to 12,
**characterized**
**in that** the egg (1) is placed in a pole-heavy fashion and the hole (2) is introduced in the region of the upwardly directed pointed pole in the case of a hole dimension of 10 mm in the case of three days of incubation, or in the case of a smaller hole, also in a possible horizontal position, if days of incubation exceed a duration of five days.

14. Method according to any one of Claims 1 to 13,
**characterized**
**in that** the hole (2) in the eggshell of the egg (1) ensures an optical access to a blood vessel (21, 27) through which blood flows.

15. Method according to any one of Claims 1 to 14,
**characterized**
**in that** the laser light (3a) is automatically focussed onto the blood vessel (21, 27).

16. Method according to any one of Claims 1 to 15,
**characterized**
**in that** the introduced power of the laser source (3) does not lead to a local or global heating of the egg (1) to above 40° Celsius.

17. Method according to any one of Claims 1 to 16,
**characterized**
**in that** the Raman scattered radiation (7) is registered using an objective lens (6) with a large numerical aperture of NA > 0.3.

18. Method according to any one of Claims 1 to 17,
**characterized**
**in that** the Raman scattered radiation (7) is recorded using a Raman fibre probe.

19. Method according to any one of Claims 11 or 12 to 18, if dependent on Claim 11,
**characterized**
**in that** the captured Raman scattered radiation (7) is fed to the spectrometer (8) via fibre lines (4b).

20. Method according to any one of Claims 1 to 19,
**characterized**
**in that** the range of 500 cm⁻¹ to 4000 cm⁻¹ of the Raman shift is used in the evaluation unit (9) for evaluating the Raman scattered radiation.

21. Method according to any one of Claims 1 to 20,
**characterized**
**in that** the sex-specific features are contained in the Raman bands of the nucleic acids, carbohydrates, lipids and proteins, which are fed to a mathematical analysis.

22. Method according to Claim 21,
**characterized**
**in that**, for the mathematical analysis, methods of supported and non-supported classification are used, wherein the sex-specific features are stored in a feature table of an evaluation unit (9).

23. Method according to any one of Claims 1 to 22,
**characterized**
**in that** the captured Raman scattered radiation (7) is corrected in the form that disturbing background signals of scattering processes are eliminated and the spectra are normalized in a predetermined form.

24. Method according to any one of Claims 1 to 23,
**characterized**
**in that** the nonlinear coherent anti-Stokes Raman scattering spectroscopy CARS is used as Raman spectroscopy, wherein the CARS scattered radiation from the blood of the blood vessel (21, 27) is recorded using the same apparatus (20) as for the spontaneous linear Raman spectroscopy with or without a fibre optic (5).

25. Method according to Claim 24,
**characterized**
**in that** the CARS signal is generated by a broadband femtosecond laser as a laser source (3), said laser serving as a pump laser and as a Stokes laser.

26. Method according to Claim 25,
**characterized**
**in that** the CARS signal is generated by two lasers, in particular by a broadband laser and a narrowband laser as laser sources (3) for using and evaluating a multiplex CARS spectroscopy.

27. Method according to any one of Claims 24 to 26,
**characterized**
**in that** the CARS spectra are used for a classification to identify the sex.

28. Method according to Claim 27,
**characterized**
**in that** the resonant component of the CARS spectrum [Im(Chi⁽³⁾)] is separated from the non-resonant component [Re(Chi⁽³⁾)] and only the resonant component is used for the classification.

29. Method according to any one of Claims 24 to 28,
**characterized**
**in that** a same classification strategy for identifying the sex is carried out for the CARS spectroscopy as for the linear Raman spectra.

30. Apparatus for determining the sex of fertilized and incubated bird eggs (1) in ovo by way of Raman spectroscopy using the methods according to any one of Claims 1 to 29, at least comprising
- an egg storage unit (16),
- a blood vessel position evaluation device (14) connected to the egg storage unit (16),
- a radiation device (19) with a light source (10) for emitting light (10a) in the visible wavelength range for identifying at least one blood vessel (21, 27), wherein the radiation device (19) transilluminates at least one part of the egg (1),
- a detector (13) for the light (10b) for identifying blood vessels (21, 27), wherein the detector (13) is connected to the blood vessel position evaluation device (14), wherein the radiation device (19) and the detector (13) form at least one vision system (19, 13),
- a hole-producing unit for creating a hole (2) in the eggshell in the vicinity of identified blood vessels (21, 27),
- a laser source (3) for emitting laser light (3a),
- an optical apparatus (5) for introducing the laser light (3a) into the hole (2) created in the eggshell of the egg (1) and for capturing the Raman scattered radiation (7) of the blood of the blood vessel (21, 27) irradiated by the laser light (3a), wherein the laser light (3a) is directed onto at least one blood vessel (21, 27) in a focussed manner,
- a spectrometer (8) for recording the Raman scattered radiation (7) of the blood of the blood vessel (21, 27) irradiated by the laser light (3a), by way of at least one optical fibre line (4b),
- a control unit (18) for xyz-positioning of the optical apparatus (5) on the hole (2) created in the eggshell of the egg (1), wherein the control unit is connected to the blood vessel position evaluation device and wherein the optical apparatus (5) is connected to the laser source (3), the spectrometer (8) and the control unit (18), and
- a sex determination evaluation unit (9) which is connected to the spectrometer (8) and the blood vessel position evaluation device (14) and which specifies the sex of the incubated bird egg (1) from the processed captured Raman scattered radiation (7) of the spectrometer (8).

31. Apparatus according to Claim 30,
**characterized**
**in that** the hole-producing unit in the form of a laser or a device for mechanical perforation is connected via a line to the blood vessel position evaluation device (14).

32. Apparatus according to Claim 30,
**characterized**
**in that** the vision system (19, 13) is a device for determining the position of the blood vessels (21, 27) with the detector (13).

33. Apparatus according to Claim 32,
**characterized**
**in that** the device for determining the position of a blood vessel (21, 27) is connected to the control unit (18) via at least one supply and signalling line (17).

34. Apparatus according to either of Claims 32 and 33,
**characterized**
**in that** the control unit (18) is embodied in the form of a coordinate positioning unit and in that the device for determining the position of a blood vessel (21, 27) is connected to a height adjustment device situated within the coordinate positioning unit (18) and the egg storage unit (16).

35. Apparatus according to Claim 34,
**characterized**
**in that** the device for determining the position of the blood vessel (21, 27), the height adjustment device and the egg storage unit (16) are connected in the blood vessel position determining unit (14) by means of the programming means for determining the position of the blood vessel (21, 27).

36. Apparatus according to any one of Claims 30 to 35,
**characterized**
**in that** the optical apparatus (5) is a flexible optical fibre.

37. Apparatus according to any one of Claims 30 to 36,
**characterized**
**in that** the radiation device (19) emits light (10a) of the visible wavelength range, at least green light (10a) for identifying at least one blood vessel (21, 27).

38. Apparatus according to any one of Claims 30 to 37,
**characterized**
**in that** the evaluation unit (9) has programming means for the sex determination evaluation of the captured Raman scattered radiation (7).

39. Apparatus according to any one of Claims 30 to 38,
**characterized**
**in that** the apparatus (5) for introducing the laser light (3a) and capturing the Raman scattered radiation (7) is connected to the control unit (18) which helps focus the laser light (3a) onto the ascertained capturable blood vessel (21, 27) in the case of the movable embryo (23).

## Revendications

1. Procédé pour la détermination in ovo par spectroscopie Raman du sexe du sexe d'oeufs d'oiseaux fécondés et incubés (1), dans lequel l'embryon (23), y compris les structures extra-embryonnaires, est mobile dans l'oeuf (1) et n'est pas encore fixé à la coquille au moment d'un enregistrement du rayonnement de diffusion Raman (7), comprenant les étapes consistant à :
- surveiller l'évolution de l'incubation jusqu'à la formation d'au moins un vaisseau sanguin (21, 27) discernable,
- au moyen d'une unité de perçage, percer un trou (2) dans la coquille dans la zone proche d'un vaisseau sanguin (21, 27) détecté,
- rechercher les vaisseaux sanguins (21, 27) qui se forment dans l'oeuf (1) au moyen d'un dispositif de rayonnement (19) destiné à éclairer coaxialement ou latéralement avec une lumière (10a) se situant dans la gamme de longueurs d'onde visible et d'un système de vision (19, 13) comportant un détecteur (13),
- positionner au moins un vaisseau sanguin (21, 27) au foyer laser d'une source laser (3) soit en déplaçant l'oeuf (1) soit en déplaçant un objectif (6) d'un dispositif (5) destiné à introduire la lumière laser (3a) et à détecter le rayonnement de diffusion Raman (7), et irradier le vaisseau sanguin avec la lumière laser (3a),
- enregistrer le rayonnement de diffusion Raman (7) du vaisseau (21, 27) irradié au moyen du dispositif (5) destiné à introduire la lumière laser (3) et à détecter le rayonnement de diffusion Raman (7), dans lequel le mouvement du vaisseau sanguin (21, 27) hors du foyer est empêché pendant l'enregistrement du rayonnement de diffusion Raman (7) par poursuite au moyen du système de vision (19, 13),
- évaluer le rayonnement de diffusion Raman (7) et déterminer le sexe de l'embryon (23) dans une unité d'évaluation (9), et
- afficher le sexe de l'embryon (23) contenu dans l'oeuf d'oiseau (1).

2. Procédé selon la revendication 1,
**caractérisé en ce que** le trou (2) est formé au pôle de plus forte courbure de l'oeuf (1) orienté vers le haut et présente un diamètre allant jusqu'à 18 mm, de préférence entre 8 mm et 15 mm, au moyen de l'unité de perçage.

3. Procédé selon la revendication 1,
**caractérisé en ce que** de la lumière blanche ou bleue et/ou verte provenant d'une source de lumière (10a) du dispositif de rayonnement (19) du système de vision (19, 13) est utilisée en tant que lumière (10a) se situant dans la gamme de longueurs d'onde visible pour surveiller l'évolution de l'incubation.

4. Procédé selon l'une des revendications 1 à 3,
**caractérisé en ce que** le procédé de détermination in ovo par spectroscopie Raman du sexe d'oeufs d'oiseaux fécondés et incubés (1) est mis en oeuvre à partir du troisième jour d'incubation.

5. Procédé de détermination in ovo par spectroscopie Raman du sexe des oeufs d'oiseaux fécondés et incubés (1), dans lequel les structures extra-embryonnaires ne sont plus librement mobiles et dans lequel une fixation de la membrane chorioallantoïdienne à la coquille commence, comprenant les étapes consistant à :
- surveiller l'évolution de l'incubation jusqu'à ce qu'une fixation de la membrane chorioallantoïdienne à la coquille commence et que des vaisseaux sanguins (21, 27) soient déjà présents,
- identifier la position des vaisseaux sanguins (24, 25, 26) par fluoroscopie avec de la lumière (10a) se situant dans la gamme de longueurs d'onde visible provenant d'une source de lumière (10) d'un système de vision (19, 13) comprenant un dispositif de rayonnement (19) et un détecteur (13),
- percer un trou (2) dans la coquille au moyen d'une unité de perçage sous forme de laser ou au moyen d'une perforation mécanique dans la région des vaisseaux sanguins (21, 27) identifiés,
- positionner les vaisseaux sanguins (21, 27) au foyer laser d'une source laser (3) en déplaçant soit l'oeuf (1) soit un objectif (6) d'un dispositif (5) destiné à introduire de la lumière laser (3a) et à détecter le rayonnement de diffusion Raman (7), et irradier les vaisseaux sanguins avec la lumière laser (3a),
- enregistrer le rayonnement de diffusion Raman (7) d'au moins un vaisseau sanguin (21, 27) irradié, dans lequel le rayonnement de diffusion Raman du sang est enregistré dans les capillaires ou dans des vaisseaux sanguins plus grands à l'intérieur ou au-dessous de la membrane chorioallantoïdienne,
- évaluer le rayonnement de diffusion Raman (7) et déterminer le sexe de l'embryon (23) dans une unité d'évaluation (9), et
- afficher le sexe de l'embryon (23) contenu dans l'oeuf d'oiseau (1).

6. Procédé selon la revendication 5,
**caractérisé en ce qu'**un trou (2) ayant un diamètre allant jusqu'à 5 mm, de préférence compris entre 0,1 et 3 mm, est réalisé au moyen de l'unité de perçage.

7. Procédé selon la revendication 5,
**caractérisé en ce que** de la lumière blanche ou bleue et/ou verte provenant de la source de lumière (10) du dispositif de rayonnement (19) du système de vision (19, 13) est utilisée en tant que lumière (10a) se situant dans la gamme de longueurs d'onde visible pour surveiller l'évolution de l'incubation.

8. Procédé selon l'une des revendications 5 à 7,
**caractérisé en ce que** le procédé de détermination in ovo par spectroscopie Raman du sexe d'oeufs d'oiseaux fécondés et incubés (1) est mis en oeuvre à partir du cinquième jour d'incubation.

9. Procédé selon l'une des revendications 1 à 8,
**caractérisé en ce qu'**une classification est effectuée selon les procédures de mesure respectives pour la reconnaissance du sexe de l'oeuf d'oiseau incubé (1) respectivement mesuré avant l'affichage du sexe.

10. Procédé selon la revendication 1 ou 5,
**caractérisé en ce que** le rayonnement de diffusion Raman (7) du sang de vaisseaux sanguins extra-embryonnaires (21) et/ou du sang de vaisseaux sanguins embryonnaires (27) est utilisé pour la détermination non invasive in ovo du sexe dans l'oeuf d'oiseau incubé (1).

11. Procédé selon l'une des revendications 1 à 10,
**caractérisé en ce que** les paramètres suivants sont utilisés pour la spectroscopie Raman :
- longueurs d'onde d'excitation de la lumière laser (3a) de la source laser (3) : > 600 nm, par exemple 633 nm pour un laser HeNe, 1064 nm pour un laser à solide Nd:YAG ou 785 nm pour un laser à diode VIS NIR,
- injecter directement la lumière laser (3a) au moyen de miroirs et/ou de fibres optiques (4a),
- effectuer des mesures de rayonnement de diffusion Raman à l'aide de dispositifs optiques (5) présentant une grande ouverture numérique tels que des objectifs de microscope (6) ou une sonde à fibres Raman,
- extraire directement le rayonnement de diffusion Raman (7) collecté au moyen de miroirs sur un spectromètre (8) ou au moyen de dispositifs de transport comportant des fibres optiques (4b),
- utiliser le spectromètre (8) sous la forme d'un spectromètre Raman dispersif et d'un spectromètre Raman à transformée de Fourier.

12. Procédé selon l'une des revendications 1 à 11,
**caractérisé en ce que**, pendant le processus de positionnement de l'oeuf d'oiseau (1), des spectres Raman renvoyés en permanence sont enregistrés et soumis à une évaluation, dans lequel une classification automatique du rayonnement de diffusion Raman (7) est effectuée sur la base de l'empreinte spectrale du sang.

13. Procédé selon l'une des revendications 1 à 12,
**caractérisé en ce que** l'oeuf (1) est placé avec son pôle pesant vers le bas et le trou (2) est percé dans la région du pôle de forte courbure orientée vers le haut pour obtenir une taille de trou de 10 mm pendant trois jours d'incubation ou, dans le cas d'un trou plus petit, également dans une position horizontale possible, pendant un nombre de jours d'incubation supérieur à cinq jours.

14. Procédé selon l'une des revendications 1 à 13,
**caractérisé en ce que** le trou (2) de la coquille de l'oeuf (1) permet d'accéder optiquement à un vaisseau sanguin (21, 27) dans lequel circule le sang.

15. Procédé selon l'une des revendications 1 à 14,
**caractérisé en ce que** la lumière laser (3a) est focalisée automatiquement sur le vaisseau sanguin (21, 27).

16. Procédé selon l'une des revendications 1 à 15,
**caractérisé en ce que** la puissance introduite de la source laser (3) ne conduit pas à un échauffement local et global de l'oeuf (1) de plus de 40° Celsius.

17. Procédé selon l'une des revendications 1 à 16,
**caractérisé en ce que** le rayonnement de diffusion Raman (7) est enregistré au moyen d'un objectif (6) à grande ouverture numérique de NA > 0,3.

18. Procédé selon l'une des revendications 1 à 17,
**caractérisé en ce que** le rayonnement de diffusion Raman (7) est reçu au moyen d'une sonde à fibres Raman.

19. Procédé selon l'une des revendications 11 ou 12 à 18, lorsqu'elle dépend de la revendication 11,
**caractérisé en ce que** le rayonnement de diffusion Raman (7) détecté est délivré au spectromètre (8) par l'intermédiaire de lignes à fibres optiques (4b).

20. Procédé selon l'une des revendications 1 à 19,
**caractérisé en ce que** la plage de 500 cm⁻¹ à 4000 cm⁻¹ du décalage Raman est utilisée pour évaluer le rayonnement de diffusion Raman dans l'unité d'évaluation (9).

21. Procédé selon l'une des revendications 1 à 20,
**caractérisé en ce que** les caractéristiques liées au sexe sont contenues dans les bandes Raman des acides nucléiques, des hydrates de carbone, des lipides et des protéines, qui sont soumises à une analyse mathématique.

22. Procédé selon la revendication 21,
**caractérisé en ce que** des procédés de classification pris en charge et non pris en charge sont utilisés pour l'analyse mathématique, dans lequel les caractéristiques liées au sexe sont enregistrées dans un tableau de caractéristiques d'une unité d'évaluation (9).

23. Procédé selon l'une des revendications 1 à 22,
**caractérisé en ce que** le rayonnement de diffusion Raman (7) détecté est corrigé de manière à ce que des signaux de fond parasites soient éliminés des processus de diffusion et que les spectres soient normalisés sous une forme prédéterminée.

24. Procédé selon l'une des revendications 1 à 23,
**caractérisé en ce que** la spectroscopie non linéaire cohérente anti-Stokes Raman CARS est utilisée en tant que spectroscopie Raman, dans lequel le rayonnement de diffusion CARS est absorbé par le sang du vaisseau sanguin (21, 27) à l'aide du même dispositif (20) que pour la spectroscopie Raman linéaire spontanée avec ou sans fibre optique (5).

25. Procédé selon la revendication 24,
**caractérisé en ce que** le signal CARS est généré par un laser femtoseconde à large bande en tant que source laser (3) qui sert de laser de pompage et de laser de Stokes.

26. Procédé selon la revendication 25,
**caractérisé en ce que** le signal CARS est généré par deux lasers, en particulier par un laser à large bande et par un laser à bande étroite en tant que sources laser (3) pour l'utilisation et l'évaluation d'une spectroscopie CARS multiplexée.

27. Procédé selon l'une des revendications 24 à 26,
**caractérisé en ce que** les spectres CARS sont utilisés pour une classification visant à reconnaître le sexe.

28. Procédé selon la revendication 27,
**caractérisé en ce que** la partie résonnante du spectre CARS [Im(Chi⁽³⁾)] est séparée de la partie non résonnante [Re(Chi⁽³⁾)] et **en ce que** seule la partie résonnante est utilisée pour la classification.

29. Procédé selon l'une des revendications 24 à 28,
**caractérisé en ce que**, pour la spectroscopie CARS, la stratégie de classification mise en oeuvre pour la reconnaissance du sexe est identique à celle qui est mise en oeuvre pour les spectres Raman linéaires.

30. Dispositif de détermination in ovo par spectroscopie Raman du sexe d'oeufs d'oiseaux fécondés et incubés (1), utilisant les procédés selon l'une des revendications 1 à 29, comprenant au moins
- une unité de stockage d'oeufs (16),
- un dispositif d'évaluation de position des vaisseaux sanguins (14) relié à l'unité de stockage d'oeufs (16),
- un dispositif de rayonnement (19) comportant une source de lumière (10) destinée à émettre une lumière (10a) se situant dans la gamme de longueurs d'onde visible afin de détecter au moins un vaisseau sanguin (21, 27), dans lequel le dispositif de rayonnement (19) irradie au moins une partie de l'oeuf (1),
- un détecteur (13) pour la lumière (10b), destiné à détecter des vaisseaux sanguins (21, 27), dans lequel le détecteur (13) est relié au dispositif d'évaluation de position des vaisseaux sanguins (14), dans lequel le dispositif de rayonnement (19) et le détecteur (13) constituent au moins un système de vision (19, 13),
- une unité de perçage destinée à percer un trou (2) dans la coquille dans la zone proche des vaisseaux sanguins (21, 27) détectés,
- une source laser (3) destinée à émettre de la lumière laser (3a),
- un dispositif optique (5) destiné à introduire la lumière laser (3a) dans le trou (2) percé dans la coquille de l'oeuf (1) et à détecter le rayonnement de diffusion Raman (7) du sang du vaisseau sanguin (21, 27) irradié par la lumière laser (3a), dans lequel la lumière laser (3a) est dirigée de manière focalisée vers au moins un vaisseau sanguin (21, 27),
- un spectromètre (8) destiné à recevoir le rayonnement de diffusion Raman (7) du sang du vaisseau sanguin (21, 27) irradié par la lumière laser (3a) par l'intermédiaire d'au moins une ligne à fibre optique (4b),
- une unité de commande (18) destinée au positionnement xyz du dispositif optique (5) sur le trou (2) percé dans la coquille de l'oeuf (1), dans lequel l'unité de commande est reliée au dispositif d'évaluation de position des vaisseaux sanguins et dans lequel le dispositif optique (5) est relié à la source laser (3), au spectromètre (8) et à l'unité de commande (18), et
- une unité d'évaluation et de détermination du sexe (9) qui est reliée au spectromètre (8) et au dispositif d'évaluation de position des vaisseaux sanguins (14) et qui indique le sexe de l'oeuf d'oiseau incubé (1) à partir du rayonnement de diffusion Raman (7) détecté et traité du spectromètre (8).

31. Dispositif selon la revendication 30,
**caractérisé en ce que** l'unité de perçage sous la forme d'un laser ou d'un dispositif de perforation mécanique est reliée au dispositif d'évaluation de position des vaisseaux sanguins (14) par une ligne.

32. Dispositif selon la revendication 30,
**caractérisé en ce que** le système de vision (19, 13) est un dispositif destiné à déterminer la position des vaisseaux sanguins (21, 27) au moyen du détecteur (13).

33. Dispositif selon la revendication 32,
**caractérisé en ce que** le dispositif de détermination de la position d'un vaisseau sanguin (21, 27) est relié à l'unité de commande (18) par au moins une ligne d'alimentation et de signalisation (17).

34. Dispositif selon l'une des revendications 32 ou 33,
**caractérisé en ce que** l'unité de commande (18) est réalisée sous la forme d'une unité de positionnement de coordonnées et **en ce que** le dispositif de détermination de la position d'un vaisseau sanguin (21, 27) est relié à un dispositif de réglage en hauteur, situé à l'intérieur de l'unité de positionnement de coordonnées (18), et à l'unité de stockage d'oeufs (16).

35. Dispositif selon la revendication 34,
**caractérisé en ce que** le dispositif de détermination de la position du vaisseau sanguin (21, 27), le dispositif de réglage en hauteur et l'unité de stockage d'oeufs (16) contenus dans le dispositif d'évaluation de position des vaisseaux sanguins (14) sont reliés par l'intermédiaire de moyens de programmation destinés à déterminer la position du vaisseau sanguin (21, 27).

36. Dispositif selon l'une des revendications 30 à 35,
**caractérisé en ce que** le dispositif optique (5) est une fibre optique flexible.

37. Dispositif selon l'une des revendications 30 à 36,
**caractérisé en ce que** le dispositif de rayonnement (19) émet de la lumière (10a) se situant dans la gamme de longueurs d'onde visible, et au moins de la lumière verte (10a) pour la détection d'au moins un vaisseau sanguin (21, 27).

38. Dispositif selon l'une des revendications 30 à 37,
**caractérisé en ce que** l'unité d'évaluation (9) comprend des moyens de programmation pour l'évaluation du rayonnement de diffusion Raman (7) détecté à des fins de détermination du sexe.

39. Dispositif selon l'une des revendications 30 à 38,
**caractérisé en ce que** le dispositif (5) d'introduction de la lumière laser (3a) et de détection du rayonnement de diffusion Raman (7) est relié à l'unité de commande (18), qui aide à focaliser la lumière laser (3a) sur le vaisseau sanguin détectable (21, 27) déterminé dans lorsque l'embryon (23) est mobile.
